# EUROPEAN PATENT SPECIFICATION

(11) **EP 0 783 520 B1**
(45) Date of publication and mention of the grant of the patent: **28.11.2001**
(21) Application number: 95933880.7
(22) Date of filing: 21.09.1995
(51) Int. Cl.: C07K 14/31, C07K 16/12, A61K 39/085, A61K 39/40

(54) **BROADLY REACTIVE OPSONIC ANTIBODIES REACTIVE WITH COMMON STAPHYLOCOCCAL ANTIGENS**
BREIT-REAKTIVE OPSONISCHE ANTIKÖRPER, DIE MIT GEMEINSAMEN STAPHYLOCOCCUS-ANTIGENEN REAGIEREN
ANTICORPS A ACTIVITE OPSONIQUE ETENDUE REAGISSANT AVEC LES ANTIGENES DU STAPHYLOCOQUE COMMUNS

(30) Priority: 21.09.1994 US 308495
(43) Date of publication of application: 16.07.1997
(73) Proprietor: HENRY M. JACKSON FOUNDATION FOR THE ADVANCEMENT OF MILITARY MEDICINE, Rockville, MD 20852 (US)
(72) Inventor: FISHER, Gerald, W., Bethesda, MD 20814-4799 (US)
(74) Representative: Grünecker, Kinkeldey, Stockmair & Schwanhäusser Anwaltssozietät
(86) International application number: US9511992
(87) International publication number: WO9609321

(56) References cited:
- WO-A-93/09811
- WO-A-93/19373
- J. MED. MICROBIOL. (1991), 35(2), 65-71, 1991 TIMMERMAN, C. P. ET AL 'Characterization and functional aspects of monoclonal antibodies specific for surface proteins of coagulase -negative staphylococci'

## Description

### Technical Field

This invention describes an isolated surface protein of a coagulase-negative staphylococcus and broadly reactive and opsonic immunoglobulin, including polyclonal and monoclonal antibodies, useful for preventing, diagnosing, and treating staphylococcal infections. This invention also describes a vaccine and methods of isolating the broadly reactive and opsonic immunoglobulin according to the invention.

### Background Art

Over the last two decades, staphylococcal infections have become important causes of human morbidity and mortality, particularly in hospitalized patients. Because of their prevalence on the skin and mucosal linings, staphylococci are ideally situated to produce infections, both localized and systemic. Debilitated or immunosuppressed patients are at extreme risk of systemic infection.

The staphylococcus species most frequently pathogenic in humans are Staphylococcus aureus and Staphylococcus epidermidis. Each species includes a number of serotypes. Both groups have developed resistance to antibiotics, the current treatment of choice.

In recent years, S. epidermidis has become a major cause of nosocomial infection in patients having treatments comprising placing implants into the body, such as cerebrospinal fluid shunts, cardiac valves, vascular catheters, and joint prostheses. S. epidermidis is also a common cause of postoperative wound infections and peritonitis in patients with continuous ambulatory peritoneal dialysis. One form of treatment for kidney failure entails the introduction of large volumes of peritoneal dialysis fluid into the peritoneal cavity, a treatment carrying a risk of frequent and recurrent infections.

Patients with impaired immunity and those receiving parenteral nutrition through central venous catheters are at high risk for developing S. epidermidis sepsis (C.C. Patrick, J. Pediatr., 116:497 (1990)). In particular, S. epidermidis has become a common cause of neonatal nosocomial sepsis, and is now the most common cause of bacteremia in the neonatal intensive care unit setting. Infections frequently occur in premature infants receiving parenteral nutrition, which can be a direct or indirect source of contamination. Such infections are difficult to treat for a variety of reasons. For example, resistance to antibiotics is common. In one study, the majority of staphylococci isolated from blood cultures of septic infants were multiply resistant to antibiotics (Fleer et al., Pediatr. Infect. Dis., 2:426 (1983)). Stimulation of the immune system provides little relief because such infants have impaired immunity resulting from deficiencies in antibodies, complement, and neutrophil function. Moreover, lipid infusion, which is now a standard ingredient of parenteral nutrition therapy, further impairs the already poor infant immune response to bacterial infection (Fischer et al., Lancet, 2:819 (1980)). Infection with S. epidermidis in these patients increases morbidity and mortality, and adds intensive care days that markedly increase medical costs.

Supplemental immunoglobulin therapy has been shown to provide some measure of protection against certain encapsulated bacteria, such as Hemophilus influenzae and Streptococcus Pneumoniae. Infants deficient in antibody are susceptible to infections from these bacteria, and thus, bacteremia and sepsis resulting from infection are common. When anti-Streptococcal and anti-Hemophilus antibodies are present, they provide protection by promoting clearance of the respective bacteria from the blood. In the case of antibody specific for staphylococcus, the potential use of supplemental immunoglobulin to prevent or treat infection has been much less clear.

Early studies of staphylococcal infections focused on the potential use of supplemental immunoglobulin to boost peritoneal defenses, such as opsonic activity, in patients receiving continuous ambulatory peritoneal dialysis. Standard intravenous immunoglobulin (IVIG) was shown to have lot to lot variability for opsonic activity to S. epidermidis (L.A. Clark and C.S.F. Easmon, J. Clin. Pathol., 39:856 (1986)). In this study, one third of the tested IVIG lots had poor opsonization with complement, and only two out of fourteen were opsonic without complement. Thus, despite the fact that the IVIG lots were made from large plasma donor pools, good opsonic antibody specific for S. epidermidis was not uniformly present. Treatment with such immunoglobulin would therefore not provide protection against Staphylococcal infection. This study did not examine whether IVIG could be used to prevent or treat S. epidermidis infections or bacterial sepsis.

Recent studies have associated coagulase-negative staphylococci, such as S. epidermidis, as the most common species causing bacteremia in neonates receiving lipid emulsion infusion (Freeman et al., N. Engl. J. Med., 323:301 (1990)). The neonates had low levels of opsonic antibody to S. epidermidis despite the fact that sera had clearly detectable levels of IgG antibodies to S. epidermidis peptidoglycan (Fleer et al., J. Infect. Dis., 2:426 (1985)). This was surprising because anti-peptidoglycan antibodies were presumed to be the principal opsonic antibodies. Thus, while suggesting that neonatal susceptibility to S. epidermidis might be related to impaired opsonic activity, these studies also suggested that many antibodies directed against S. epidermidis are not opsonic and would not be capable of providing protection when given passively to neonates. Moreover, the antigens responsible for inducing opsonic antibodies were not identified.

Recently, an antigen binding assay was used to analyze IgG antibody to S. epidermidis in patients with uncomplicated bacteremia and in patients with bacteremia and endocarditis (Espersen et al., Arch. Intern. Med., 147:689 (1987)). This assay used an ultrasonic extract of S. epidermidis to identify S. epidermidis specific IgG. None of the patients with uncomplicated bacteremia had IgG antibodies specific for S. epidermidis. These data suggest that IgG does not provide effective eradication of S. epidermidis from the blood. In addition, 89% of bacteremic patients with endocarditis developed high levels of IgG to S. epidermidis. In these patients, IgG was not protective since high levels of IgG antibody were associated with serious bacteremia and endocarditis. Based on these studies, the protective role of IgG in S. epidermidis sepsis and endocarditis was questionable, especially in the presence of immaturity, debilitation, intralipid infusion, or immunosuppression.

The role of antibody in immunity to S. epidermidis has also been studied in animal models (Kojima et al., J. Infect. Dis., 162:435-441 (1990); and Yoshida et al., J. Appl. Bacteriol., 47:299-301 (1979)). Animal studies that demonstrated immunoglobulin protection against staphylococcal infections have shown strain specificity by enzyme-linked immunosorbent assays (ELISA). These studies utilized normal adult mice having a mature immune system in protection studies, and therefore do not mimic the disease observed in humans. Studies using mature animals with normal immunity typically comprise administering to the animals unusually virulent strains or overwhelming-challenge doses of bacteria. This does not mimic infection in humans because human patients are generally immunologically immature or debilitated. Human patients can also have somewhat indolent infections with low virulence pathogens, such as S. epidermidis, with death usually attributable to secondary complications rather than the bacterial infection. Models using unusual strains or overwhelming bacterial doses generally induce rapid fulminant death.

These factors are important since antibodies generally work in concert with the host cellular immune system (neutrophils, monocytes, macrophages, and fixed reticuloendothelial system). The effectiveness of antibody therapy may therefore be dependent on the functional immunologic capabilities of the host. To be predictive, animal models must closely mimic the clinical condition in which the infection occurs and capture the setting for therapy.

Prior animal studies have yielded inconsistent results. One animal model used an unusually virulent strain of S. epidermidis. Infected mature mice developed 90 to 100% mortality within 24 to 48 hours (Yoshida et al., Japan. J. Microbiol., 20:209 (1976)). Antibody to S. epidermidis surface polysaccharide was protective in these mice, with protection occurring for an IgM fraction but not an IgG fraction (K. Yoshida and Y. Ichiman, J. Med. Microbiol., 11:371 (1977)).

This model presents a pathology very different from that typically seen in infected patients. Intraperitoneally-challenged mice developed symptoms of sepsis within minutes of receiving the injection and died in 24 to 48 hours. This pathology is not observed in staphylococcus-infected humans. The highly virulent strain of S. epidermidis may represent an atypical type of infection. Moreover, isolates of S. epidermidis from infected humans did not kill mice in this model.

In 1987, animal studies were extended to include the evaluation of antibodies in human serum against selected virulent strains of S. epidermidis (Ichiman et al., J. Appl. Bacteriol., 63:165 (1987)). In contrast to previous data, protective antibody was found in the IgA, IgM, and IgG immunoglobulin fractions. A definitive role for any single class of immunoglobulin (IgG, IgM, IgA) could not be established.

In this animal model, mortality was determined for normal adult mice. Death was considered to be related to the effect of specific bacterial toxins, not bacteremia sepsis (Yoshida et al., Japan J. Microbiol., 20:209 (1976)). Most clinical isolates did not cause lethal infections, and quantitative blood cultures were not done. This study provided little insight as to whether antibody could successfully prevent or treat S. epidermidis sepsis in immature or immunosuppressed patients.

In a later animal study, serotype specific antibodies directed against S. epidermidis capsular polysaccharides were tested. Results showed that serotype-specific antibodies were protective, but that each antibody was directed against one particular serotype as measured by ELISA (Ichiman et al., J. Appl. Bacteriol., 63 :165 (1987)). Protection was equally serotype specific. Protection against heterologous strains did not occur. In addition, it was concluded that protection was afforded by the IgM antibody.

In short, there has been no compelling evidence that IVIG, which contains only IgG, could be effective to treat and prevent S. epidermidis infections or sepsis, particularly where patients are immature or immune suppressed, or where multiple S. epidermidis serotypes are involved. Thus, for example, a recent and extensive review of the pathogenesis, diagnosis, and treatment of S. epidermidis infections does not include immunoglobulin as a potential prophylactic or therapeutic agent (C.C. Patrick, J. Pediatr., 116:497 (1990)).

An animal model that mimics human S. epidermidis infections, particularly for humans that are immature or immune suppressed, has been described in WO-A-9319373. These patients have low levels of complement as well as impaired neutrophil and macrophage function. Thus, even if opsonic activity of immunoglobulin may appear adequate under optimal conditions in vitro, protection may not occur in patients such as newborn babies or cancer patients. Previous models were unsatisfactory in that they used animals which did not possess similar risk factors as the typical high-risk human patient.

Although coagulase negative staphylococci (CNS) are significant as nosocomial pathogens, no effective method to prevent CNS infections has been developed. The current preferred treatment of choice for the prevention and cure of staphylococcal infections in humans is antibiotic therapy. Although new antibiotics are constantly being developed, it has become increasing clear that antibiotic therapy alone is insufficient. Data regarding passive vaccinations with immunoglobulin is at best unclear. The animal models on which this therapy has been attempted bear little relationship to human infections and as yet, have produced no definitive solutions. In summary, there is a need in the art for an effective treatment for staphylococci.infections.

WO-A-9319373 relates to the isolation by generally known methods of an antigen which, upon introduction into a host, generates an antibody that is useful to prevent, diagnose, or treat staphylococcus infections. However, the antigen has not been physically characterized.

### Disclosure of the Invention

The present invention overcomes the problems and disadvantages associated with current strategies and relates to a new therapy for the treatment and prevention of staphylococcal infections. This invention describes broadly reactive opsonic immunoglobulin reactive with common staphylococcal antigens from which vaccines, pharmaceutical compositions, and diagnostic aids can be created for the treatment and prevention of staphylococcal infections in both man and animals.

In particular, the invention describes a common surface protein present on several S. epidermidis strains having different serotypes. Although this surface protein is from a single S. epidermidis strain, it induces broadly reactive and opsonic antibodies. Thus, the protein is useful for screening plasma to make opsonic immunoglobulin that is broadly reactive across all three serotypes of S. epidermidis, and for a vaccine to induce active immunity to S. epidermidis.

The invention also describes an isolated opsonic antibody against a surface protein of a coagulase-negative staphylococcus, wherein the protein induces antibodies that are broadly reactive against staphylococci. This antibody can be a monoclonal or polyclonal antibody. In a preferred embodiment the surface protein is from S. epidermidis and has a molecular weight of about 45,000 to 50,000 dalton, and an iselectric focusing point of approximately pH 4.5.

Further, the invention describes methods of isolating broadly reactive and opsonic immunoglobulin useful for the treatment and prevention of a staphylococcus infection and a vaccine comprising a therapeutically effective amount of a surface protein from a coagulase-negative staphylococcus, which protein induces antibodies that are broadly reactive against staphylococci, and a pharmaceutically acceptable carrier. The invention also includes a pharmaceutical composition for the treatment or prevention of staphylococcus infections comprising an opsonic antibody against a surface protein from a coagulase-negative staphylococcus, wherein the antibody is broadly reactive against staphylococci, and a pharmaceutically acceptable carrier.

Other objects and advantages of the present invention are set forth in the following description. The accompanying drawings and tables, which constitute a part of the disclosure, illustrate and, together with this description, explain the principle of the invention.

### Brief Description of the Drawings

- Figure 1:: Antibody titers of human plasma tested for binding to S. epidermidis serotypes I, II, III, and Hay.
- Figure 2:: Pre- and post-immunization ELISA titers of sera from rabbits immunized with a TCA-extracted antigens of S. epidermidis Hay (ATCC 55133) tested for binding to S. epidermidis serotypes I, II, III, and Hay.
- Figure 3 :: Pre- and post-immunization ELISA titers of sera from rabbits immunized with a whole cell preparation of S. epidermidis Hay (ATCC 55133) tested for binding to S. epidermidis serotypes I, II, III, and Hay.
- Figure 4:: Effect of absorption of immunoglobulin with S. epidermidis on opsonization. Neutrophil mediated opsonization assay of S. epidermidis, S. aureus, and Streptococcus agalactiae organisms using immunoglobulin selected for the ability to bind to a preparation of S. epidermidis, and selected immunoglobulin preabsorbed with a preparation of S. epidermidis. Negative control is neutrophils plus complement alone.
- Figure 5:: Opsonic antibody response (opsonic activity) to S. epidermidis Serotypes I, II, III, and Hay measured as percent bactericidal response to rabbit serum pre- and post-immunization with a TCA-extracted antigen preparation of S. epidermidis Hay (ATCC 55133).
- Figure 6:: Opsonic antibody response (opsonic activity) to S. epidermidis Serotypes I, II, III, and Hay measured as percent bactericidal response to rabbit serum pre- and post-immunization with a whole cell preparation of S. epidermidis Hay (ATCC 55133).
- Figure 7:: Opsonic activity of pre- and post-immunization serum with TCA-extracted antigens or whole cell preparation of S. epidermidis Hay (ATCC 55133) against S. aureus type 5. Opsonic assays were calculated using two dilutions of the reaction mixture prior to subculturing on to solid agar.
- Figure 8:: Effect of high titer vs. low titer IVIG to S. epidermidis on clearance of S. epidermidis from the blood of animals with S. epidermidis sepsis. Bacteremia levels of S. epidermidis were measured in samples of blood from suckling rats treated with either high titer immunoglobulin, selected for the ability to bind to a preparation of S. epidermidis, or unselected low-titer immunoglobulin.
- Figure 9 :: Effect of directed (selected high-titer) immunoglobulin and saline injections on survival in suckling rats treated with intralipid plus S. epidermidis.
- Figure 10:: Effect of directed (selected high-titer) immunoglobulin, directed immunoglobulin preabsorbed with a preparation of S. epidermidis, and saline injections on survival in suckling rats treated with intralipid plus S. epidermidis.
- Figure 11:: Effect of directed (selected high-titer) immunoglobulin, directed immunoglobulin preabsorbed with a preparation of S. epidermidis, and saline injections on bacteremia levels in the blood of suckling rats treated with intralipid plus S. epidermidis.
- Figure 12:: Relationship between opsonic activity measured in vitro and survival in the suckling rat lethal animal model with directed (selected high-titer) immunoglobulin, unselected low-titer immunoglobulin, directed immunoglobulin preabsorbed with a preparation of S. epidermidis, and saline.
- Figure 13:: Samples of S. epidermidis were analyzed by two-dimensional gel electrophoresis. A 45-50,000 dalton protein which focuses at a pH of approximately 4.5 was identified on all S. enidermidis serotypes.

### Best Mode for Carrying Out the Invention

The present invention describes the identification, preparation, and isolation of immunoglobulin and antigen useful for preventing, diagnosing, or treating staphylococcal infections. In particular, the invention provides a single screen with a staphylococcal organism with the proper antigens that will identify broadly reactive and opsonic antibodies to staphylococcus that are pathogenic to humans.

In one aspect, the present invention provides broadly opsonic antibodies of S. epidermidis, protective across all three serotypes. Such antibodies are induced by a surface protein. Antibodies against this protein are useful opsonins to enhance phagocytosis and eradication of bacteria from a host. The protein can also be used as a tool for screening plasma or immunoglobulins (polyclonal or monoclonal) useful for passive immunotherapy to prevent or treat S. epidermidis infections. In addition, this protein is useful for active immunization to induce protection against S. epidermidis by vaccination. A particularly useful surface protein has a molecular weight of approximately 45-50 Kd.

### Methods to Identify the Immunoglobulin of the Invention

To identify these broadly opsonic and reactive antibodies, the invention provides a method comprising an assay to identify immunoglobulin (from pooled or individual samples of plasma, serum, whole blood, or tissue, such as placenta) reactive with a preparation of a staphylococcal organism having broadly reactive constituent antigens to identify broadly reactive and opsonic immunoglobulin.

The preparation is a cell surface extract. It is also preferred that the preparation is from S. epidermidis Hay (ATCC 55133) or any other organism bearing the antigens that induce broadly reactive antibodies.

A suitable preparation may be prepared by isolating a culture of bacterial cells of S. epidermidis Hay (ATCC 55133), suspending the isolated cells in a mixture comprising a solution of trichloroacetic acid (TCA), stirring the mixture at approximately 4°C, centrifuging the mixture and saving the resulting supernatant. This is followed by combining the supernatant with an alcohol, preferably absolute ethanol, incubating the alcohol-supernatant combination at approximately 4°C to precipitate a preparation, and finally isolating the precipitated preparation.

### The assays

### Binding assays

A preferred assay employs an in vitro assay that identifies opsonic antibody, such as a binding assay or opsonization assay. In a preferred binding assay, immunoglobulin is reacted with a preparation of a staphylococcal organism. The binding assay is preferably an enzyme-linked immunosorbent assay (ELISA) or a radioimmunoassay (RIA), but may also be an agglutination assay, a coagglutination assay, a colorimetric assay, a fluorescent binding assay, or any other suitable binding assay. The assay can be performed by competitive or noncompetitive procedures with results determined directly or indirectly.

The staphylococcus preparation may be fixed to a suitable solid support, such as a glass or plastic plate, well, bead, micro-bead, paddle, propeller, or stick. The solid support is preferably a titration plate. The fixed preparation is incubated with immunoglobulin, which is isolated or in a biological fluid, and the amount of binding determined. A positive reaction occurs when the amount of binding observed for the test sample is greater than the amount of binding for a negative control. A negative control is any sample known not to contain antigen-specific immunoglobulin. Positive binding may be determined from a simple positive/negative reaction or from the calculation of a series of reactions. This series may include samples containing measured amounts of immunoglobulin that specifically bind to the fixed antigen, creating a standard curve from which the amount of antigen-specific immunoglobulin in an unknown sample can be determined. Alternatively, antibody can be fixed to a solid support and immunoglobulin identified by its ability to bind a bacterial preparation bound to the fixed antibodies.

### Opsonization assays

An opsonization assay can be a colorimetric assay, a chemiluminescent assay, a fluorescent or radiolabel uptake assay, a cell-mediated bactericidal assay, or any other appropriate assay which measures the opsonic potential of a substance and identifies broadly reactive immunoglobulin. In an opsonization assay, the following are incubated together: an infectious agent, a eukaryotic cell, and the opsonizing substance to be tested, or an opsonizing substance plus a purported opsonizing enhancing substance. Preferably, the opsonization assay is a cell-mediated bactericidal assay. In this in vitro assay, the following are incubated together: an infectious agent, typically a bacterium, a phagocytic cell, and an opsonizing substance, such as immunoglobulin. Although any eukaryotic cell with phagocytic or binding ability may be used in a cell-mediated bactericidal assay, a macrophage, a monocyte, a neutrophil, or any combination of these cells, is preferred. Complement proteins may be included to observe opsonization by both the classical and alternate pathways.

The opsonic ability of immunoglobulin is determined from the amount or number of infectious agents remaining after incubation. In a cell-mediated bactericidal assay, this is accomplished by comparing the number of surviving bacteria between two similar assays, only one of which contains the purported opsonizing immunoglobulin. Alternatively, the opsonic ability is determined by measuring the numbers of viable organisms before and after incubation. A reduced number of bacteria after incubation in the presence of immunoglobulin indicates a positive opsonizing ability. In the cell-mediated bactericidal assay, positive opsonization is determined by culturing the incubation mixture under appropriate bacterial growth conditions. Any significant reduction in the number of viable bacteria comparing pre- and post-incubation samples, or between samples which contain immunoglobulin and those that do not, is a positive reaction.

### Clearance/protective assays

Another preferred method of identifying agents for the treatment or prevention of a staphylococcal infection employs a lethal model of staphylococcus sepsis that measures clearance and protection. Such agents can be immunoglobulin or other antimicrobial substances. This model can also be used for screening anti-Staphylococcal drugs.

A particularly useful animal model comprises administering an antibody, an immune suppressant, and a staphylococcal organism to an immature animal, followed by evaluating whether the antibody reduces mortality of the animal or enhances clearance of the staphylococcal organism from the animal. This assay may use any immature animal, including the rabbit, the guinea pig, the mouse, the rat, or any other suitable laboratory animal. The suckling rat lethal animal model, comprising an immature animal further immunosuppressed by the administration of an immune suppressant, is most preferred.

An immune suppressant is any substance which impairs the immune system of the animal to which it is administered, and is selected from the group consisting of steroids, antiinflammatory agents, prostaglandins, cellular immune suppressants, iron, silica, particles, beads, lipid emulsions, and any other effective immune suppressant. Preferably, the immune suppressant is cyclosporin, dexamethasone, triamcinolone, cortisone, prednisone, ibuprofen, or any other related compound or combination of compounds. More preferably, the immune suppressant is a lipid emulsion, and the lipid emulsion of choice is intralipid. When the pharmaceutical composition is immunoglobulin, the assay measures the clearance potential of the administered immunoglobulin.

Clearance is evaluated by determining whether the pharmaceutical composition enhances clearance of the infectious agent from the animal. This is typically determined from a sample of biological fluid, such as blood, peritoneal fluid, or cerebrospinal fluid. The infectious. agent is cultured from the biological fluid in a manner suitable for growth or identification of the surviving infectious agent. From samples of fluid taken over a period of time after treatment, one skilled in the art can determine the effect of the pharmaceutical composition on the ability of the animal to clear the infectious agent. Further data may be obtained by measuring over a period of time, preferably a period of days, survival of animals to which the pharmaceutical composition is administered. Typically, both sets of data are utilized. Results are considered positive if the pharmaceutical composition enhances clearance or decreases mortality. In situations in which there is enhanced organism clearance, but the test animals still perish, a positive result is still indicated.

### Method of Isolating the Immunoglobulin

Still another embodiment of the present invention is isolated immunoglobulin. The assay may be any type of immunological assay, such as a binding assay, opsonization assay, or clearance assay as set forth above.

A method of isolating broadly reactive and opsonic immunoglobulin according to the invention comprises:
(a) providing a sample of immunoglobulin;
(b) assaying the immunoglobulin for the presence of an antibody against a surface protein of a coagulase-negative staphylococcus, wherein the protein induces antibodies that are broadly reactive against staphylococci; and
(c) isolating immunoglobulin having a high titer of said antibody;
wherein the presence of antibody against said protein is indicative of the usefulness of the biological fluid for the treatment and prevention of the staphylococcal infections.

In another method according to the invention, the immunoglobulin is assayed for the presence of antibody against a Serotype II capsular polysaccharide antigen of a coagulase-negative staphylococcus, wherein the antigen induces antibodies broadly reactive against staphylococci; subsequently the immunoglobulin is assayed for the presence of antibody against a surface protein antigen according to the invention, and immunoglobulin having a high titer of said antibody is isolated.

Still another method of isolating broadly reactive and opsonic immunoglobulin according to the invention requires the immunoglobulin to be assayed for the presence of antibody against a Serotype II coagulase-negative staphylococcus bearing a surface protein antigen, or a combination thereof, wherein the antigens induce opsonic antibodies that are broadly reactive against staphylococci. Preferably, the coagulase-negative staphylococcus is Staphylococcus epidermidis or preferably strain Hay deposited at the ATCC under Accession No. 55133.

Isolated immunoglobulin can be obtained from pooled or single units of blood, plasma, sera, or tissue, such as placenta, or from any immunoglobulin preparation derived therefrom, such as intravenous immunoglobulin (IVIG). Procedures for the isolation of immunoglobulin are well-known to those of ordinary skill in the art. Exemplary procedures are described in Protein Purification: Principles and Practice (R.K. Scopes, Springer-Verlag, New York, 1987), incorporated by reference.

Isolated immunoglobulin, including polyclonal antibodies, monoclonal antibodies, or a mixture thereof, can be one or more antibodies of any isotype, including IgG, IgM, IgD, IgA, or IgE, but is preferably IgG. Procedures for the identification and isolation of a particular fraction or isotype of antibody are well-known in the art. Exemplary methods are taught in Current Protocols in Immunology (Coligan et al., eds., John Wiley & Sons, New York, 1991), incorporated by reference. The present invention also includes methods for making these antibodies.

Methods for making polyclonal and monoclonal antibodies are known in the art. Certain methods, by way of example, are described in Antibodies: A Laboratory Manual (E. Harlow and D. Lane, Cold Spring Harbor Lab., 1988), incorporated by reference.

Monoclonal IgG antibodies are preferable. IgG isotype antibodies can be made by isolating an IgG-producing hybridoma cell or by genetic manipulation. Also preferred is a method of producing purely or partly human monoclonal antibodies. Nonhuman or partly human antibodies may be made more human by chimerization or genetic manipulation.

The present invention includes an antigen binding site attached to the structural portion of an antibody molecule, or attached to another protein reactive in an assay with a preparation of a staphylococcal organism having broadly reactive surface antigens.

### Isolated surface protein

Another embodiment of the present invention is isolated surface protein of a coagulase-negative staphylococcus, wherein said protein induces antibodies that are broadly reactive against staphylococci.

In a preferred embodiment, the isolated surface protein is the 45-50 Kd surface protein of S. epidermidis. Although any organism bearing the antigens that induce the broadly reactive antibodies of the invention can be the source of the isolated surface protein, a preferred source is Serotype II S. epidermidis Hay (ATCC 55133).

Methods of macromolecular purification include filtration, fractionation, precipitation, chromatography, affinity chromatography, HPLC, FPLC, electrophoresis, and any other suitable separation technique. Methods for the purification of proteins are well-known in the art.

The surface protein may be purified, substantially purified, or partially purified. Exemplary protein purification methods are described in Proteins: Structures and Molecular Properties (T.E. Creighton, W.H. Freeman and Co., New York, 1984); and Carbohydrate Analysis: A Practical Approach, 2nd Edition (D. Rickwood, ed., IRL Press, Oxford England, 1984), incorporated by reference. Exemplary methods for the identification, production, and use of synthetic antigens are described in Laboratory Techniques in Biochemistry and Molecular Biology: Synthetic Polypeptides as Antigens (R.H. Burden and P.H. Knippenberg, eds., Elsevier, New York, 1988), incorporated by reference.

The present invention also encompasses a recombinant surface protein. The DNA sequence of the gene coding for the isolated surface protein can be identified, isolated, cloned, and transferred to a prokaryotic or eukaryotic cell for expression by procedures well-known in the art. For example, procedures are generally described in Current Protocols in Molecular Biology (Ausubel et al., eds., John Wiley & Sons, 1989), incorporated by reference.

Upon introduction into a host, isolated surface protein generates a polyclonal or monoclonal antibody broadly reactive and opsonic in an assay with a staphylococcal organism preparation. Preferably, the staphylococcal organism is-Serotype II S. epidermidis Hay (ATCC 55133).

### Pharmaceutical Compositions

The present invention also discloses a pharmaceutical composition comprising an opsonic antibody against a surface protein from a coagulase-negative staphylococcus, wherein the antibody is broadly reactive against staphylococci, including polyclonal and monoclonal antibodies, and a pharmaceutically acceptable carrier.

Pharmaceutically acceptable carriers can be sterile liquids, such as water, oils, including petroleum oil, animal oil, vegetable oil, peanut oil, soybean oil, mineral oil, sesame oil, and the like. With intravenous administration, water is a preferred carrier. Saline solutions, aqueous dextrose, and glycerol solutions can also be employed as liquid carriers, particularly for injectable solutions. Suitable pharmaceutical carriers are described in Remington's Pharmaceutical Sciences, 18th Edition (A. Gennaro, ed., Mack Pub., Easton, Pa., 1990).

### Method of Evaluating Efficacy

A method for evaluating the efficacy of a pharmaceutical composition useful for treating an infectious agent comprises administering a pharmaceutical composition, an immune suppressant, and an infectious agent to an immature animal, preferably an immune suppressed suckling rat. This is followed by evaluating whether the pharmaceutical composition reduces mortality of the animal or enhances clearance of the infectious agent from the animal. This method can be used where the infectious agent is a bacterium, preferably a gram positive bacterium, a parasite, a fungus, or a virus.

Immune suppressants are described above. The pharmaceutical composition is administered prophylactically for evaluating the efficacy of the pharmaceutical composition in enhancing resistance to an infectious agent or therapeutically for evaluating the efficacy of the pharmaceutical composition comprising the broadly reactive and opsonic immunoglobulin or antimicrobial agent in directly killing the infectious agent or enhancing the immune response of a multiply immunocompromised and lethally infected animal to fight off the infection.

### Diagnostic Kit

An isolated opsonic antibody according to the invention can be used to produce a diagnostic kit and aid for detecting a staphylococcal infection. The diagnostic aid comprises broadly reactive immunoglobulin (such as polyclonal or monoclonal antibodies) and a sample of biological fluid containing or suspected of containing antigen or antibody to staphylococcus.

A method for detecting staphylococcal infection in an animal comprises adding a biological sample containing or suspected of containing antibody specific for staphylococcus to isolated antigen, followed by determining the amount of binding between the antibody and the antigen. Alternatively, this method comprises adding a biological sample comprising or suspected of comprising staphylococcus antigen to immunoglobulin specific for a preparation of a staphylococcal organism, followed by determining the amount of binding between antigen present in the sample and the immunoglobulin. The immunoglobulin can be polyclonal or monoclonal antibody, but is preferably monoclonal antibody.

Exemplary methods are taught in Immunology: A Synthesis (E.S. Golub, Sinauer Assocs., Inc., Sunderland, Ma., 1987), incorporated by reference.

In one example, the diagnostic aid can be used to identify in a laboratory isolate human pathogenic staphylococcus. Staphylococci can be grouped into two groups based on a coagulase test: coagulase-negative, of which S. epidermidis is the most common pathogen, and coagulase-positive, of which S. aureus is the most common pathogen. Most, if not all, human pathogenic S. epidermidis are Serotype II coagulase-negative. Preliminary data shows that human pathogenic staphylococci react with antisera to the Serotype II capsular polysaccharide of S. epidermidis. Thus, the Serotype II capsular antigen appears to be a human virulence marker.

A laboratory isolate can be any organism isolated by microbiological techniques from, for example, a human source, an animal source, or other source. Laboratory isolates may also contain nonpathogenic contaminants.

The diagnostic aid is useful for determining if staphylococci, particularly coagulase-negative Serotype II staphylococci, present in the isolate are pathogenic for humans. The methods described above for performing assays are applicable therefor.

Another use of the diagnostic aid is for identifying staphylococci and antigens thereof in body fluids of an animal. For example, a diagnostic aid reactive with coagulase-negative pathogenic staphylococci can be used to identify the presence of pathogenic staphylococci or antigens thereof in body fluids. Body fluids that can be tested include, but are not limited to, cerebrospinal fluid, blood, peritoneal fluid, and urine. The diagnostic aid is employed according to the methods described above. Detection using this diagnostic aid can be performed in cases of actual, suspected, acute, or chronic infection with staphylococci. Likewise, antigens from pathogenic staphylococcal organisms can be used to detect antibody to pathogenic organisms in blood and body fluids.

### Method of Detecting a Pharmaceutical Composition

An isolated surface protein and an isolated opsonic antibody according to the invention can be used in a method for detecting a pharmaceutical composition in a biological sample. When a pharmaceutical composition comprises immunoglobulin, the method comprises adding a biological sample containing the pharmaceutical composition to isolated surface protein, followed by determining the amount of binding between the pharmaceutical composition and the isolated surface protein. Alternatively, when the pharmaceutical composition comprises isolated surface protein, this method comprises adding a biological sample comprising the pharmaceutical composition to an antibody specific for the pharmaceutical composition, followed by determining the amount of binding between the pharmaceutical composition and the antibody.

These methods may be used, inter alia, to determine the pharmacokinetics of the pharmaceutical composition comprising broadly reactive and opsonic immunoglobulin. With this information, better care can be provided by determining the best dosage regimen and course of treatment with a pharmaceutical composition.

The following examples set forth the various aspects of the invention.

### Example 1

The purpose of this example is to demonstrate that large immunoglobulin pools can not ensure the presence of a high titer of antibody to S. epidermidis.

IgG fractions of standard intravenous immunoglobulin (IVIG) were used in experiments to represent large immunoglobulin pools. Preparations of various pools of IgG from several companies were analyzed for comparison (Gamimmune, Cutter Labs., Inc., Berkeley, California: Sandoglobuin, Sandoz, East Hanover, N.J.; Gammagard, Hyland, Los Angeles, California; Polygam, American Red Cross, Washington, D.C.).

Samples from each of these pools, and one sample from an individual patient (SAM), were tested for binding in an enzyme-linked immunosorbent assay (ELISA) against a preparation of S. epidermidis. Although any S. epidermidis strain can be used, the experiments used Hay, a clinical strain isolated from the blood of a child with S. epidermidis sepsis. This strain is on deposit at the American Type Culture Collection (ATCC) under Accession No. 55133.

Briefly, a culture of S. epidermidis (Hay, ATCC 55133) was grown to log phase (18-36 hours) at 37°C in 1600 ml aliquots of tryptic soy broth (Difco Labs., Detroit, Michigan). The culture was centrifuged at 5000 rpm for 10 minutes and the cell buttons resuspended in a small volume (10-25 mls) of 2% TCA at pH 2.0. The TCA suspensions were combined and stirred overnight at 4°C, and the next day, the combined suspension was centrifuged at 5000 rpm for 10 minutes, the supernatants aspirated and saved, and the cell buttons discarded. Supernatants were combined with four volumes of absolute ethanol and stored overnight at 4°C. This solution was centrifuged at 2500 rpm for 10 minutes, the supernatants aspirated and discarded, and the antigen precipitates resuspended in saline and cultured to ensure sterility. Saline suspensions were lyophilized and stored at 4°C.

TCA-extracted antigen for ELISA testing was made from each serotype by dissolving 1.0 mg of lyophilized extract in 40 mls of coating buffer. Coating buffer was prepared by combining 1.59 g Na₂CO₃, 2.93 g NaHCO₃, and 0.2 g NaN₃ and adding distilled water to a final volume of 1000 mls. This solution was adjusted to a pH of 9.6. One hundred microliter aliquots of the antigen-containing solution were added to each well of 96-well microtiter plates, using separate plates for each serotype. Plates were incubated overnight at 4°C, after which wells were emptied and rinsed four times with PBS-Tween. PBS-Tween was prepared by combining 8.0 g NaCl, 0.2 g KH₂PO₄, 2.9 g Na₂HPO₄, 0.2 g KCl, 0.2 g NaN₃, and 0.5 mls of Tween-20, and adding distilled water to a final volume of 1000 mls. The solution was adjusted to a pH of 7.4. Samples of 100 *µ*ls from each pool of immunoglobulin were added to wells. Plates containing antisera were incubated at 4°C for two hours, after which the plates were again emptied and rinsed four times with PBS-Tween. A 1/400 dilution of stock alkaline phosphatase-conjugated goat anti-rabbit IgG (Sigma Chem. Co., St. Louis, Mo.) was prepared in PBS-Tween. Aliquots of 40 *µ*ls were added to each well of the microtiter plates and the plates were incubated for two hours at 4°C. The plates were again emptied and rinsed four times with PBS-Tween. A l mg/ml solution of p-nitrophenyl phosphate (Sigma Chem. Co., St. Louis, Mo.) was prepared in diethanolamine buffer and 100 *µ*l aliquots of this solution were added to each well of the microtiter plates. Diethanolamine buffer was prepared by combining 97 mls diethanolamine and 0.2 g NaN₃, and adding distilled water to a final volume of 1000 mls. The solution was adjusted to a pH of 9.8. The plates were incubated at 37°C for two hours. Absorbance was measured at 405 nm using the Multiskan® MCC/340 instrument (Flow Labs., Lugano, Switzerland).

**TABLE I**

| **Antigen Binding Activity of Human Immunoglobulin for Staphylococcus epidermidis (ATCC 55133)** | | |
|---|---|---|
| Immunoglobulin; Source Lot | | Optical Density |
| Baxter | 609 | 0.707 |
| Baxter | 224 | 0.648 |
| | | |
| Sandoz | 163 | 0.731 |
| Sandoz | 110 | 0.786 |
| Sandoz | 069 | 0.901 |
| | | |
| Cutter | 40P07 | 1.014 |
| Cutter | 2801 | 0.666 |
| Cutter | 40R09 | 1.026 |
| | | |
| | SAM | 1.002 |

As indicated in Table I, there was a marked difference in the binding activity of each pool tested. Most samples contained low levels of antibody to S. epidermidis. Interestingly, a sample with one of the lowest activities (2801) and the sample with the highest (40R09) are from the same source, Cutter Laboratories. Among the higher binding pools, 069 and 40R09 were obtained from separate companies.

This data indicates that no single method of immunoglobulin preparation, *i.e.*, unscreened plasma or IgG pool, can ensure the presence of a high titer of antibody to S. epidermidis, despite the fact that each of the tested pools represent very large collections of human sera. Variations in the content of reactive antibody occurred between preparations prepared by the same company and between lots of the same preparation, indicating that all immunoglobulin pools are distinct and that differences in the content of a specific-identifiable antibody can be striking.

### Example 2

In a second immunoglobulin binding study, random samples of plasma from almost one hundred human patients were screened in an ELISA. Antibody titers to four different strains of S. epidermidis were determined. One strain was obtained from the American Type Culture Collection, Rockville, Maryland (ATCC 31423; Serotype 1). Two others, Serotypes 2 and 3, were provided by Dr. Y. Ichiman of the St. Marianna University School of Medicine, Japan, described in Y. Ichiman, J. Appl. Bacteriol., 56:311 (1984).

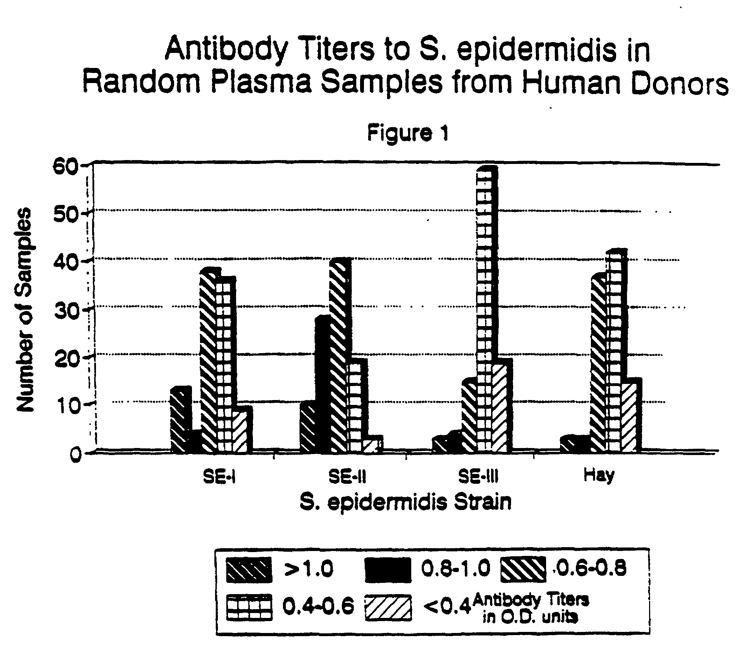

Preparations of each strain were prepared as before. The ELISA was performed as previously described, except that 40 *µ*ls of each sample were used. As shown in Figure 1, a significant number of samples contained antibody to each strain of S. epidermidis, including the clinical strain, Hay (ATCC 55133).

This data indicates that although there was a great deal of variability in binding, cross-reacting antibodies may be present within a single sample.

### Example 3

Pooled immunoglobulin could contain antibodies against a variety of S. epidermidis strains, which would mimic a single broadly reactive antibody. Therefore, studies were performed by immunizing animals with a single S. epidermidis strain to determine if exposure to this single strain would induce broadly reactive antibody.

Rabbits were immunized with either a heat-killed whole cell or TCA-extracted antigens of S. epidermidis. TCA-extracted antigens of S. epidermidis were prepared as described. One milligram of this preparation was dissolved in 1.0 ml of normal saline, and administered intramuscularly to New Zealand White rabbits. Following a one week rest, a second 1.0 ml dose was given. A final dose given one week later completed the primary immunization series. An identical third (P3), fourth (P4), or fifth (P5) course of immunization can be included, and additional booster series can be used to further elevate specific antibody levels. Further booster immunizations were given at additional intervals.

The bacterial whole cell vaccine was prepared as follows. Tryptic soy broth was inoculated with S. epidermidis (Hay, ATCC 55133) and incubated for three hours at 37°C. A 20 ml aliquot of this preparation was centrifuged at 3000 rpm for 10 minutes, the supernatant discarded, and the cell pellet resuspended in normal saline. A second washing with saline was carried out following a repeat centrifugation. The final suspension was prepared in saline to yield a total volume of 10 mls. The bacteria were heated to 56°C for 60 minutes to produce the heat killed whole cell vaccine, which was cultured to ensure sterility.

One milliliter of this whole cell preparation was administered intravenously to New Zealand White rabbits daily for five days. After a one week rest, the rabbits were again immunized daily for five days. An identical third (P3), fourth (P4), or fifth (PS) course of immunization can be included, and additional booster series can be used to further elevate specific antibody levels. Further booster immunizations were given at additional intervals.

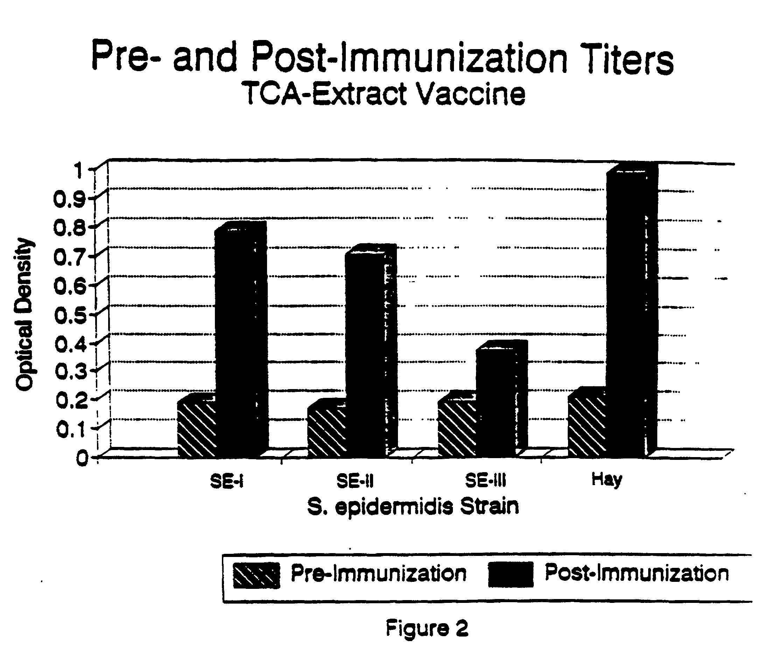

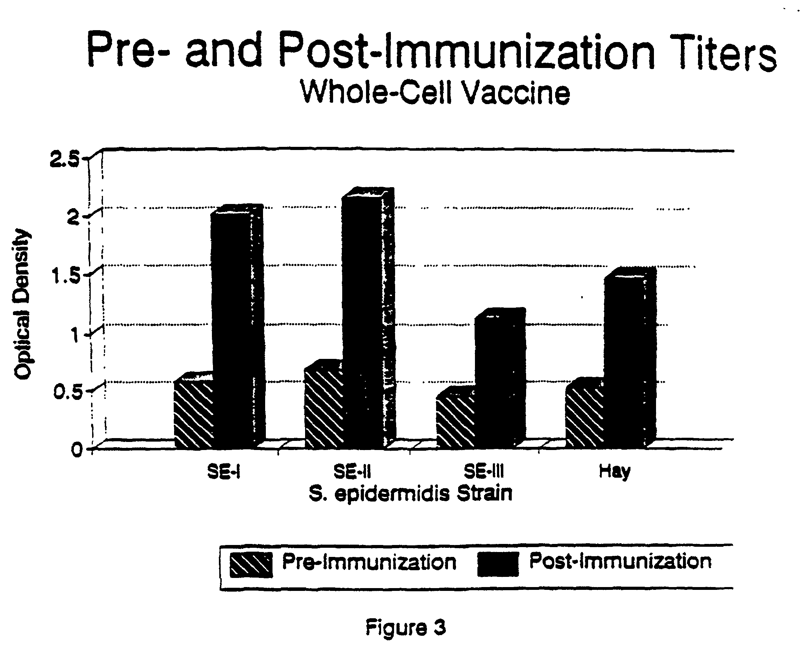

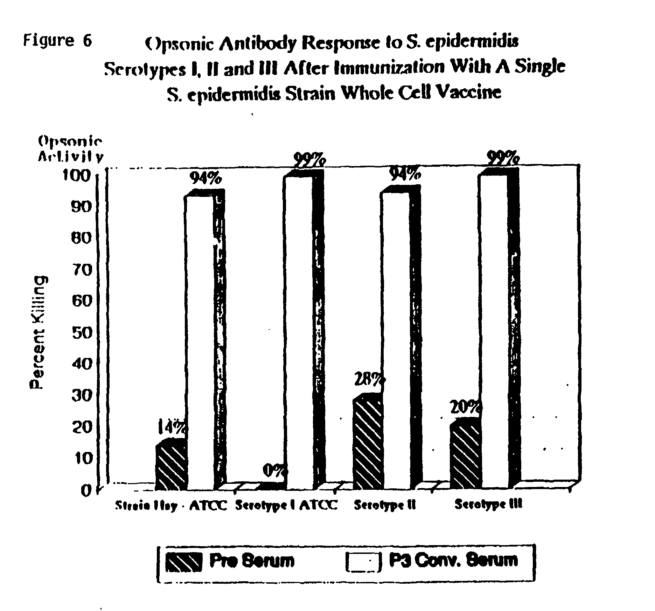

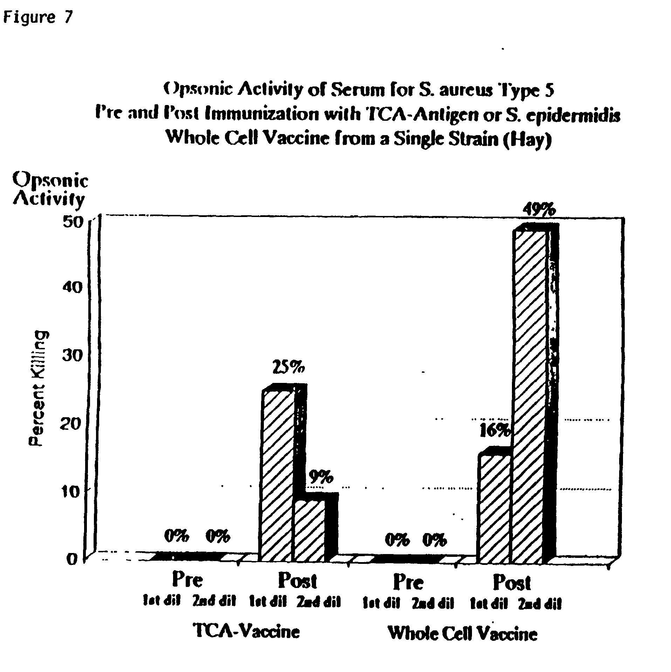

Sera obtained after immunization with the whole cell preparation showed a marked increase in antibodies to S. epidermidis, while the overall magnitude of the immune response was reduced in serum obtained after TCA-extracted antigen immunization (Figures 2 and 3). Sera induced by animals immunized with TCA-extracted antigens or whole cell vaccine produced broadly reactive antibodies to Serotypes I, II, and III of S. epidermidis plus the vaccine strain, S. epidermidis Hay (ATCC 55133), as determined by ELISA. Moreover these post-immunization antisera were broadly opsonic. (Figures 6 and 7).

As the animals were exposed only to a single strain, and as there was an equivalent background level of binding before immunization, it is clear that both preparations of S. epidermidis produced antibodies reactive with multiple S. epidermidis serotypes.

The lethal, neonatal S. epidermidis sepsis model shows that opsonic antibodies enhance clearance of bacteria from the blood and improve survival. Thus, consistent with the findings of K. Yoshida and Y. Ichiman, antibodies to Serotype II S. epidermidis capsule are protective against Serotype II polysaccharide-bearing bacteria. The results in the lethal sepsis model show that protection is mediated through opsonic antibodies that enhance bacterial clearance from the blood.

### Example 4

All antibodies, even those directed against a given organism, may not enhance immunity and provide enhanced protection from infection. Stated differently, antibodies which bind an antigen may not necessarily enhance opsonization or clearance of the organism from the infected animal and enhance survival. Therefore, a neutrophil mediated bactericidal assay was used to determine the functional activity of antibody to S. epidermidis.

Neutrophils were isolated from adult venous blood by dextran sedimentation and Ficoll-Hypaque® density centrifugation. Utilizing a microtiter plate assay requiring a total volume of 0.1 ml/well, washed neutrophils (approximately 10⁶ cells) were added to round-bottomed microtiter wells, along with approximately 3 x 10⁴ mid-log phase bacteria (S. epidermidis Hay, ATCC 55133). Newborn rabbit serum (10 *µ*ls), screened to assure absence of antibody to S. epidermidis, served as a source of active complement. Forty microliters of 5% standard IVIG (or serum) were added at various dilutions, and the microplates were incubated at 37°C with constant, vigorous shaking. Samples of 10 *µ*ls were taken from each well at zero time and after 2 hours of incubation, diluted, vigorously vortexed to disperse the bacteria, and cultured on blood agar plates overnight at 37°C to quantitate the number of viable bacterial colonies. Controls consisted of neutrophils plus complement alone, and neutrophils plus complement. The opsonic activity determined as percent bacterial killing is calculated using the formula ([number bacteria (zero time - 2 hours)]/[number bacteria at zero time]) x 100.

**TABLE IIa**

| **Opsonic Activity of Pools of Human Immunoglobulin for Staphylococcus epidermidis** | |
|---|---|
| Immunoglobulin | Opsonic Activity (Percent) |
| Cutter | |
| 801 | 45 |
| 926 | 0 |
| P07 | 92 |
| R09 | 90 |
| | |

| Sandoz | |
|---|---|
| 100 | 3 |
| 163 | 8 |
| 110 | 12 |
| 069 | 15 |
| | |

| Baxter | |
|---|---|
| 807 | 23 |
| 609 | 18 |
| 224 | 54 |
| | |
| 004 | 54 |
| | |
| SAM | 97 |
| control* | 0 |

| | |
|---|---|
| (* = neutrophil plus complement alone) | |

Opsonic activity varied from 0% to 23% and from 90% to 97% in the samples. As was observed in the binding assay, no correlation could be drawn between preparative techniques used and functional activity observed. However, some of the immunoglobulin having a high degree of binding in Table I (O.D. > 1.0), also had a high level of opsonic activity in Table IIa (e.g., 40P07, 40R09 and SAM).

Opsonophagocytic bactericidal activity of ≥ 90% (≥ 1 log reduction in bacteria over 2 hours) was arbitrarily chosen to indicate high opsonic activity.

**TABLE IIb**

| **Opsonophagocytic Bactericidal Activity of Pools of Human Immunoglobulin (IVIG) Preparations for 3 Staphylococcus epidermidis Strains** | | | |
|---|---|---|---|
| Immunoglobulin Source Lot | Opsonophagocytic Bactericidal Activity | | |
| | Strain 31432 | Strain 35984 | Strain 55133 |
| Cutter | | | |
| 801 | 45% | 59% | 49% |
| 926 | 0 | 66% | 58% |
| P07 | 92% | 88% | 92% |
| R09 | 90% | 89% | 79% |
| | | | |

| Sandoz | | | |
|---|---|---|---|
| 100 | 3% | 0 | 0 |
| 163 | 8% | 0 | 0 |
| 110 | 12% | 8% | 0 |
| 069 | 15% | 23% | 43% |

| Baxter | | | |
|---|---|---|---|
| 807 | 23% | 62% | 48% |
| 609 | 18% | 62% | 48% |
| 224 | 54% | 53% | 0 |
| (* IVIG was tested at a final concentration of 20 mg/ml IgG.) | | | |

These results show that only some of the immunoglobulin that bound to TCA-extracted antigens of S. epidermidis promoted phagocytosis and killing of S. epidermidis. Thus, for the first time using in vitro screening assays, it is possible to select immunoglobulin having high levels of antibody for S. epidermidis and having reliable levels of antibody to prevent and treat S. epidermidis infections.

### Example 5

It was important to determine if the opsonic antibodies for S. epidermidis were specifically directed against serotype specific S. epidermidis antigens or if the opsonic antibodies were directed against common staphylococcal antigens. To investigate these alternatives, selected high-titer immunoglobulin was preabsorbed with a preparation of S. epidermidis Hay (ATCC 55133) and tested for opsonic activity against three different gram positive cocci.

Absorbing bacteria were grown overnight on blood agar plates, scraped from the plates, suspended in normal saline, and pelleted in 0.5 ml microfuge tubes to one-fifth the volume of the tube. After adding 0.4 mls of immunoglobulin to each, the tubes were vortexed and rotated at a slow speed on an end-over-end tumbler (Fisher Scientific Co., Pittsburgh, Pa.) at 4°C overnight. Bacteria were sedimented the following day in a microfuge tube and the supernatant was removed and filtered through a 0.2 *µ*m membrane filter. The sterile immunoglobulin, containing no detectable S. epidermidis binding antibodies, was used either directly or after storage at 70°C.

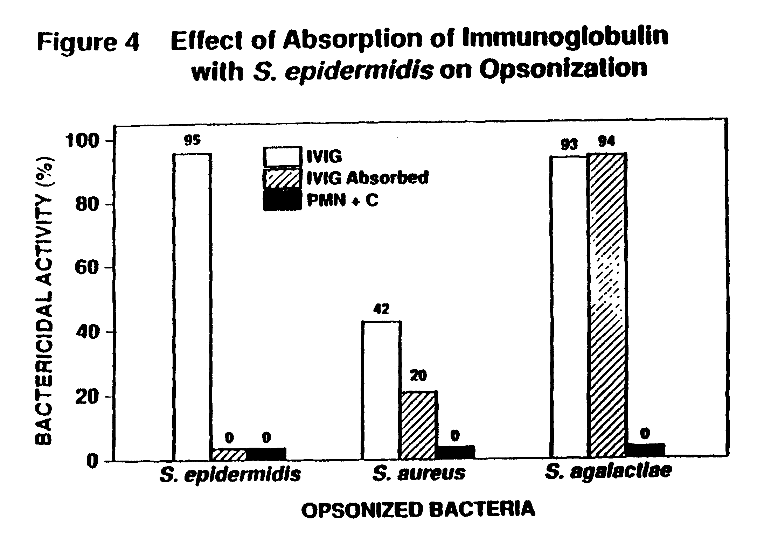

Selected high-titer immunoglobulin (directed immunoglobulin) showed opsonization of the two species of staphylococcus, S. epidermidis and S. aureus, and the one species of Streptococcus tested, S. agalactiae (Figure 4). With selected immunoglobulin preabsorbed with a preparation of S. epidermidis, opsonic activity to S. epidermidis was completely removed (95% to 0% bactericidal activity). However, opsonic activity against Streptococcus agalactiae, a different genus, was not diminished (93% to 94%). Surprisingly, a reduction in opsonic activity was observed for S. aureus (kindly provided by Dr. Mendiola of the Walter Reed Army Medical Center), present in the selected immunoglobulin at about half the level as antibody activity to S. epidermidis.

The results also suggest the existence of antibodies to antigens shared by S. epidermidis and S. aureus. Therefore, this selected immunoglobulin preparation promoted opsonization by common anti-staphylococcal antibodies that can be identified by absorption with S. epidermidis.

In the absence of antibody, there was no bactericidal activity demonstrated against any of the bacteria (neutrophil plus complement alone). These results indicate that the anti-staphylococcal antibodies are directed against key staphylococcal antigens that provide both specific protection against S. epidermidis and broad protection against other staphylococcus serotypes and species.

### Example 6

Opsonic activity was determined for serum from rabbits immunized with TCA-extracted antigens of S. epidermidis and a whole cell preparation of S. epidermidis.

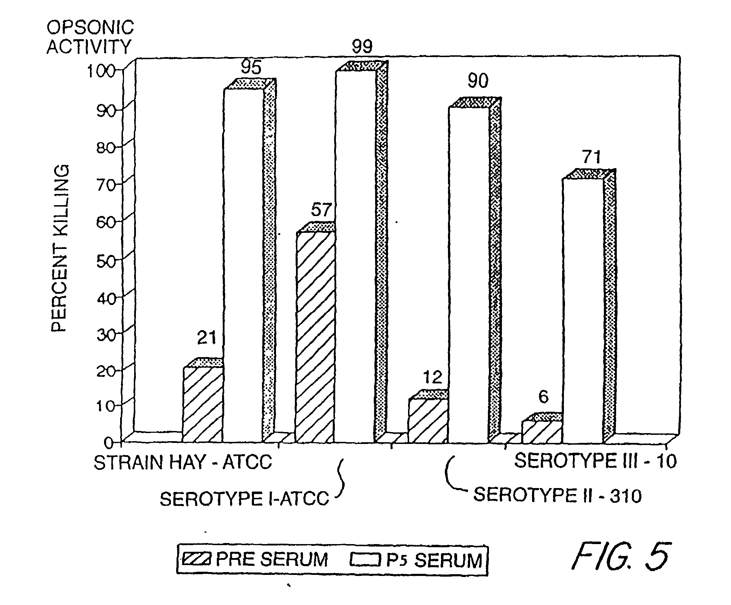

Rabbits were immunized with either TCA-extracted antigens or whole cell preparation of S. epidermidis Hay (ATCC 55133). Sera was collected as before and tested for opsonizing activity against Serotype I, II, and III strains of S. epidemidis, and S. epidermidis Hay (ATCC 55133) in the neutrophil mediated bactericidal assay. As shown in Figures 5 and 6, both TCA-extracted antigens and whole cell preparations induced an antibody response with very high opsonic activity against all three serotypes. Although pre-vaccinated serum using the TCA-extracted antigens did show some activity against Serotype I (Figure 5), opsonizing activity nearly doubled after inoculation, indicating that staphylococcal common antibodies were indeed responsible.

These data show that antibodies to S. epidermidis capsular antigens are important for immunity, and that one or more antigens may be antigenically similar between different serotypes.

### Example 7

The opsonizing activity of vaccinated rabbit sera was again determined using S. aureus Serotype 5 as the test bacterium (Figure 7). Overall opsonizing activity against S. aureus was not as high as activities observed against strains of S. epidermidis, but serum samples from immunized animals did provide significant activity as compared to unvaccinated samples.

This data indicates that opsonizing antibodies to S. epidermidis are also protective against S. aureus, and again suggests that these antibodies may be directed against one or more staphylococcal common antigens.

### Example 8

Many bacteria, including S. epidermidis, are not pathogenic in normal humans. However, in infants with an immature immune system and in individuals with an impaired immune system, S. epidermidis can cause sepsis and even death. Therefore, in any animal model of sepsis it is critical to include these factors. By utilizing an animal with an immature immune system and subjecting the animal to immunological suppressant, sepsis in human patients can be studied.

To demonstrate that IVIG with opsonic antibody directed against S. epidermidis could provide protection from lethal S. epidermidis sepsis, a suckling rat lethal animal model was developed. Suckling rats infected with 5 x 10⁷ S. epidermidis subcutaneously developed bacteremia within two hours, and cleared over 72 hours (Table III).

**TABLE III**

| **Induction of Bacteremia and Sepsis in Suckling Rats After Challenge with Staphylococcus epidermidis (ATCC 55133)** | | | |
|---|---|---|---|
| Time Post Infection | Number Bacteremic* | Percent Bacteremic | Bacteria/Ml Blood (geometric mean) |
| 2 hours | 8/8 | 100 | 3.8 x 10² |
| 4 hours | 7/8 | 87.5 | 1.3 x 10² |
| 6 hours | 8/8 | 100 | 7.5 x 10² |
| 14 hours | 6/8 | 75 | 8.8 x 10¹ |
| 18 hours | 3/8 | 37.5 | 0.5 x 10¹ |
| 22 hours | 0/8 | 0 | 0 |

| | | | |
|---|---|---|---|
| (* 8/8 (100%) infected rat pups survived) | | | |

All of the animals cleared bacteremia within 72 hours after infection (Table III), suggesting that under normal circumstances, neonatal immunity, although impaired, can eventually control S. epidermidis. However, some studies in rats infected with S. epidermidis shortly after birth have demonstrated that a lethal infection can still develop (data not shown).

### Example 9

The effect of intralipid on S. epidermidis mortality in suckling rats was assayed. Wistar rats were injected with intralipid, an immune suppressant, just after birth. Animals were administered intralipid beginning on day two of life. Two doses were administered each day for two days. With the final dose of intralipid, animals were also given selected immunoglobulin or saline. After this final dose the animals were infected by subcutaneous injection with a preparation of S. epidermidis Hay (ATCC 55133). Blood samples were subcultured onto plates to ensure that bacteremia was caused by staphylococcus and to follow clearance after therapy. All animals were followed for five days to determine survival.

**TABLE IV**

| **Animal Model: The Effect of Intralipid Dose on Staphylococcus epidermidis Mortality in Suckling Rats** | | | | |
|---|---|---|---|---|
| Intralipid Dose* | Infected | | Survival Control | |
| 4 gm/kg | 10/10 | 100% | 7/7 | 100% |
| 8 gm/kg | 10/13 | 76% | 9/9 | 100% |
| 12 gm/kg | 7/12 | 58% | 11/11 | 100% |
| 16 gm/kg | 6/13 | 46% | 11/11 | 100% |
| *16 gm/kg | 2/6 | 33% | 5/5 | 100% |

| | | | | |
|---|---|---|---|---|
| * = Intralipid was given at a dose of 4 gm/kg (up to 4 doses over 2 days) IP with the final dose given on day 3 of life, approximately 30-60 minutes prior to infection with S. epidermidis. | | | | |

Animals receiving only S. epidermidis successfully overcame infection and survived. Only those animals treated with intralipid prior to infection showed a marked decrease in their ability to resist S. epidermidis.

The administration of lipid emulsion simulates lipid administration commonly given to neonates, previously shown to impair bacterial clearance (Fischer et al., Lancet, 2:819 (1980)). In contrast to the results of Example 8, where all the pups survived treatment, at a dosage of ≥ 8 gm/kg prior to S. epidermidis challenge, survival decreased in direct proportion to the quantity of lipid administered.

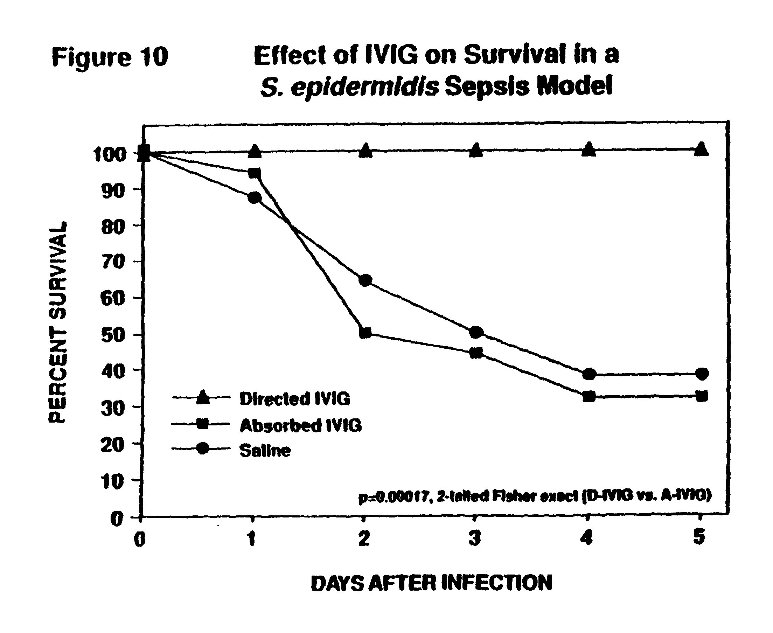

Control animals given lipid emulsion without infection suffered no apparent effects. This model may be very relevant for newborn babies, since lipid emulsion therapy has previously been associated with S. epidermidis bacteremia in neonates (Freeman et al., Eng. J. Med., 323:301-308 (1990)). For IVIG treatment studies, all animals received 16 gm/kg lipid emulsion before S. epidermidis challenge. All pups treated with IVIG containing ≥ 90% opsonic activity for S. epidermidis survived (Figure 10). Those treated with absorbed IVIG had a mortality similar to those treated with saline placebo (Survival 11/11 [100%], 9/22 [41%], and 8/15 [53%], respectively; p < .001 IVIG vs. Absorbed IVIG by Fisher's exact test).

### Example 10

The effectiveness of selected high-titer (directed) immunoglobulin in providing protection against a lethal infection of S. epidermidis Hay (ATCC 55133) was determined in the suckling rat lethal animal model.

Two day old Wistar rats were given two 0.2 ml intraperitoneal injections of 20% intralipid. The next day, animals were again given the same series of injections of 20% intralipid plus immunoglobulin or serum from vaccinated animals. After the last injection, approximately 5 x 10⁷ cells of S. epidermidis Hay (ATCC 55133) were injected subcutaneously at the base of the tail. Mortality was determined for five days.

**TABLE Va**

| **Effectiveness of Immunoglobulin Directed Against Staphylococcus epidermidis in Providing Protection from Lethal Infection** | | | |
|---|---|---|---|
| Immunoglobulin | Treated | Died | Mortality |
| Exp. #1 | | | |
| | | | |
| 40R09 | 24 | 0 | 0 % |
| Standard | 20 | 4 | 20 % |
| Control-untreated | 13 | 7 | 54 % |
| -uninfected | 11 | 0 | 0 % |
| | | | |

| Exp. #2 | | | |
|---|---|---|---|
| 40R09 | 13 | 2 | 8 % |
| Vaccine Induced | 11 | 2 | 18 % |
| Control - saline | 19 | 11 | 42 % |

Directed immunoglobulin, selected for the ability to bind to or opsonize a preparation of S. epidermidis (lot No. 40R09), provided complete protection from lethal infection in an immune-impaired lethal animal model. These results are identical to the results obtained from uninfected animals. Unselected low-titer immunoglobulin (also called standard immunoglobulin) demonstrated 20% mortality, and other controls were as expected. Untreated and uninfected animals had greater than 50% mortality.

In a second, similar experiment, directed high-titer human immunoglobulin and vaccine induced high-titer rabbit serum, both strongly protective, produced nearly identical results. In contrast, a saline control had over 40% mortality.

Overall, these data suggest that antibodies directed against S. epidermidis are protective in the suckling rat lethal animal model.

### Example 11

Several IVIG lots from various suppliers were further analyzed to determine whether screening IVIG for S. epidermidis-specific opsonic antibody could identify IVIG that would consistently enhance protection (Table Vb). IVIG with >90% bactericidal opsonic activity against S. epidermidis was compared with IVIG lots with <50% opsonic activity for S. epidermidis or with saline. Survival was significantly increased in animals receiving IVIG with ≥90% opsonic activity when compared with animals receiving IVIG with ≤50% opsonic activity or saline.

**TABLE Vb**

| **Effect of IVIG on Survival in a Neonatal Staphylococcus epidermidis Sepsis Model** | | | | |
|---|---|---|---|---|
| Study Group | Animals Treated | Animals Survived | Percent Survival | Significance (Chi Square) |
| HIV* (high titer) | 217 | 165 | 76% | p<0.0001 |
| IVIG** (low titer) | 194 | 94 | 48% | p = 0.41 |
| Saline | 56 | 23 | 41% | |

| | | | | |
|---|---|---|---|---|
| *IVIG: opsonic 2 different products, with each lot having >90% activity for S. epidermidis | | | | |
| **IVIG: <50% 4 different products (5 lots), with each lot having opsonic activity for S. epidermidis | | | | |

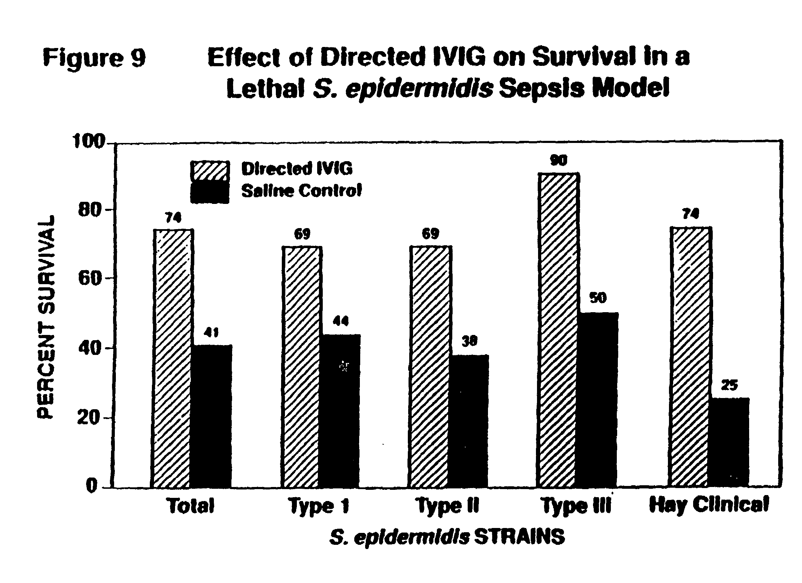

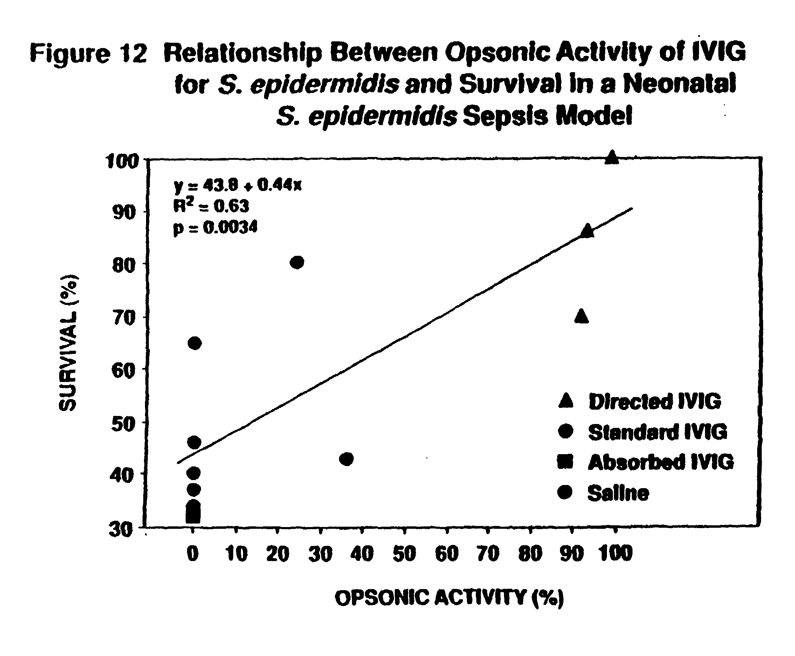

A significant relationship (p = 0.0034) was demonstrated by linear regression analysis between survival following infection with S. epidermidis and the S. epidermidis opsonic activity of the preparation administered (Figure 12). Further studies were performed to determine if IVIG was protective against multiple S. epidermidis serotypes. An IVIG lot with ≥90% opsonic activity to S. epidermidis (clinical strain) provided enhanced survival in the neonatal suckling rat model for all serotype strains and the clinical isolate Hay (Figure 9).

### Example 12

Immunoglobulin bound to a preparation of S. epidermidis in an ELISA assay, and opsonized S. epidermidis organisms in the cell mediated bactericidal assay (directed immunoglobulin), were tested for their capacity to promote clearance of S. epidermidis in the suckling rat model.

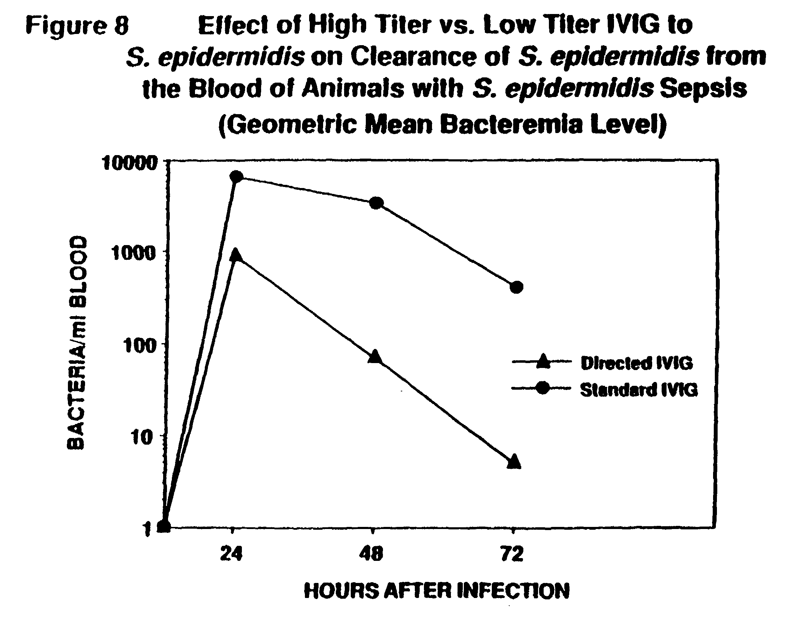

Blood samples were taken from infected animals at regular intervals (Figure 8). Only directed immunoglobulin previously identified in an ELISA or opsonic assay decreased levels of bacteria over the course of treatment. These animals showed increased survival rates in Table Va. Immunoglobulin which did not opsonize or bind to a preparation of S. epidermidis did not promote clearance of bacteria from the blood of infected animals.

### Example 13

Antibody to S. epidermidis was analyzed in the suckling rat lethal animal model for the ability to enhance clearance and provide protection against an international geographically diverse group of S. epidermidis strains (Figure 9).

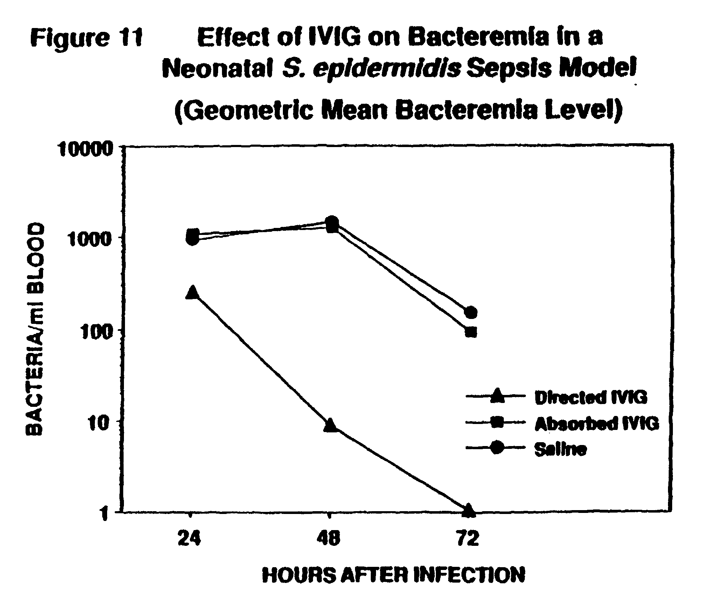

Directed immunoglobulin enhanced survival was tested against S. epidermidis Hay (ATCC 55133, Serotype II), a prototype laboratory strain (ATCC 31423, Serotype I), and two distinct Japanese strains (Serotypes II and III). Directed immunoglobulin preabsorbed against a preparation of S. epidermidis showed no increase in survival (Figure 10). Bacteria counts from blood samples taken during the study also showed that directed immunoglobulin rapidly cleared staphylococcus bacteremia. Rats treated with saline or preabsorbed immunoglobulin had persistent bacteremia and increased mortality (Figure 11).

To determine if survival was related to functional anti-staphylococcus activity of antibody, immunoglobulin preparations with various levels of opsonophagocytic bactericidal activity for S. epidermidis (directed immunoglobulin) were compared with saline and preabsorbed immunoglobulin (which had no bactericidal activity for S. epidermidis).

A significant relationship was observed between opsonophagocytic bactericidal activity of antibody and survival in staphylococcus sepsis (Figure 12). While saline, standard immunoglobulin, and preabsorbed directed immunoglobulin provided similarly poor protection (each had little or no opsonophagocytic bactericidal antibody), the unabsorbed directed immunoglobulin provided uniformly good survival. These results indicate that opsonic anti-staphylococcus antibodies are associated with survival.

### Example 14

Previous reports have suggested that there are multiple S. epidermidis serotypes. In addition, there are many other coagulase negative staphylococci besides S. epidermidis. For efficacious broadly reactive antibody, antibody ideally should cover human pathogenic coagulase negative staphylococci. Many coagulase negative staphylococci, however, rarely if ever cause infections in humans. Thus, it is important to determine if broadly reactive antibodies are capable of binding all human pathogenic coagulase negative bacteria.

Rabbits were immunized with staphylococci of one of three S. epidermidis strains (ATCC 31432, S. epidermidis 360, and S. epidermidis 10). S. epidermidis (ATCC 31432) is Serotype I, S. epidermidis 360 and S. epidermidis Hay (ATCC 55133) are Serotype II, and S. epidermidis 10 is Serotype III. The antisera were identified as follows: anti-I was raised against strain ATCC 31432; anti-II was raised against strain S. epidermidis 360; and anti-III was raised against strain S. epidermidis 10.

Coagulase negative staphylococci isolated from patients were speciated and characterized as pathogens if in a given patient there were >2 positive cultures from normally sterile sites (cultures obtained at different times or from different sites). These cultures were then reacted with rabbit antisera (anti-I, anti-II, and anti-III) in an ELISA assay.

### ELISA Assay:

***Preparation of ELISA plates:*** 100 λ aliquots of S. epidermidis extracted antigens were added to wells of 96 well microassay plates (Nunclon® , Nunc, Denmark), and stored overnight at 4°C. Wells are gently washed with Tween (0.5 ml Tween 20/1 deionized H₂O) prior to use.

Preparation of antisera: Rabbit antisera anti-I, anti-II, and anti-III were produced according to the general method of Fischer et al., J. Exper. Med., 148:776-786 (1978). Antiserum preparations were then diluted 100 fold in PBS-Tween prior to use. Further serial dilutions were also carried out in PBS-Tween. The rabbit antisera (anti-I, anti-II, and anti-Ill) were prepared further by absorption with the two heterologous strains to remove common staphylococcal antibodies not specific to one of the strains.

Analysis of antibody reactivity: Microassay plates were prepared using 40 λ of antisera at several dilutions (1/100 to 1/12800). Antisera was added to the appropriate wells of the microassay plate. Normal saline, used as a control, was similarly diluted. Plates were incubated at 4°C for two hours. Alkaline phosphatase-conjugated goat anti-rabbit IgG (Sigma, St. Louis, MO) was prepared in a 1/400 dilution with PBS-Tween, and 40 λ of this preparation was then added to each well in appropriate columns. To a single column of wells, only PBS-Tween was added. Plates were again incubated at 4°C for two hours.

4-nitrophenyl phosphate was used as substrate for the enzymatic reaction, and was prepared by dissolving a 5 mg substrate tablet (10⁴ phosphate substrate tablets, Sigma) in 5 ml of 10% diethanolamine buffer (see below). 100 λ of this substrate preparation was then added to each well as appropriate after incubation at 37°C, and absorbance was then measured at 405 nm at 120 minutes using the Titertek® Multiskan MCC/340 instrument (Flow Laboratories, Lugano, Switzerland).

***Preparation of reagents:*** Preparation of buffers from the methods of Voller et al, Bull. W.H.O., 53:55-64 (1976).

**TABLE VI**

| **Preparation of Reagents** | | | | | |
|---|---|---|---|---|---|
| Coating buffer (pH 9.6) | | PBS-Tween (pH 7.4) | | Diethanolamine buffer (pH 9.8) | |
| Na₂CO₃ | 1.59 g | NaCl | 8.0 g | Diethanolamine | 97 ml |
| NaHCO₃ | 2.93 g | KH₂PO₄ | 0.2 g | NaN₃ | 0.2 g |
| NaN₃ | 0.2 g | Na₂HPO₄ | 2.9 g | H₂O | to 1000 ml |
| H₂O | 1000 ml | KCl | 0.2 g | | |
| | | Tween 20 | 0.5 ml | | |
| | | NaN₃ | 0.2 g | | |
| | | H₂O | 1000 ml | | |

The results of these studies are shown in Table VII. Three coagulase negative staphylococci, in addition to S. epidermidis, were identified as human pathogens. Each of the pathogenic staphylococci reacted with rabbit antisera obtained after immunization with a single S. epidermidis strain, Serotype II S. epidermidis 360. Absorbing the antiserum from S. epidermidis 360 with the other two S. epidermidis strains (used to produce the other antisera but not this antisera) did not remove the staphylococcal-reactive antibodies induced by S. epidermidis 360.

Antisera raised against the other strains, however, did not react to any of the pathogenic strains after absorption with Serotype II S. epidermidis 360. In addition, Serotype II S. epidermidis Hay (ATCC 55133) reacted with the broadly reactive antisera, further showing that antigens from this organism bind antibodies in the broadly reactive antisera.

**TABLE VII**

| **Human Pathogenic* Coagulase Negative Staphylococci Reactive with Antibodies from Immunization with a Single Coagulase Negative Staphylococcus** | | |
|---|---|---|
| Organism | No. Isolated | Positive Reaction |
| S. epidermidis | 16 (57%) | 16/16 (100%) |
| S. haemolyticus | 8 (29%) | 8/8 (100%) |
| S. hominis | 3 (11%) | 3/3 (100%) |
| S. simulans | 1 ( 3%) | 1/1 (100%) |
| S. warneri | 0 | - |
| S. capitis | 0 | - |

| | | |
|---|---|---|
| *Isolates were selected only from patients with ≥2 positive cultures from sterile sites (different times or different sources). | | |

Although S. epidermidis has been divided into 3 serotypes (Y. Ichiman and K. Yoshida, J. Appl. Bacteriol., 51:229 (1981)), it has not been shown that pathogenicity is associated with any specific strain or strains using mouse virulence testing (Y. Ichiman, J. Appl. Bacteriol., 56:311 (1984)). The results presented in this example demonstrate that all of the pathogenic human coagulase negative staphylococci reacted with antibodies elicited by immunization with a single Serotype II S. epidermidis strain.

The immunizing Serotype II S. epidermidis 360 strain and Serotype II S. epidermidis Hay (ATCC 55133) are both reactive with antisera to which all of the human pathogens reacted. The results demonstrate that antigens on the surface of the human pathogens, the immunizing S. epidermidis 360 and S. epidermidis Hay (ATCC 55133), are similar, and that the antigens are important virulence markers on many coagulase negative staphylococci, including S. epidermidis, S. hemolyticus, S. hominis, and S. simulans.

Antibodies to a single S. epidermidis strain with the proper constituents (such as S. epidermidis Hay (ATCC 55133)) can confer broad protection against coagulase negative staphylococci. Antibodies raised against these antigenic determinants are useful for distinguishing between pathogenic and nonpathogenic staphylococci in laboratory isolates. Such antibodies are also useful for detecting pathogenic staphylococci and antigens thereof, or antibodies directed against pathogenic staphylococci and antigens thereof, in mammalian body fluids such as cerebrospinal fluid, blood, peritoneal fluid, and urine.

In addition, the antigens that elicit these antibodies are useful for screening immunoglobulin for broadly opsonic and protective antibodies. The antigens are also useful for producing staphylococcal vaccines.

### Example 15

The present example determines the total protein composition of the various serotypes of S. epidermidis, and identifies proteins reactive with opsonic rabbit antisera.

Mouse antibody to capsular polysaccharide has been shown to be protective for homologous S. epidermidis serotypes, but not for heterologous serotypes (Yoshida et al., J. Appl. Bacteriol., 51:229 (1981)). Protection with human serum was also related to homologous, but not heterologous anti-capsular polysaccharide antibodies (Ichiman et al., J. Appl. Bacteriol., 63:165 (1987)).
Although the mechanism of homologous protection was unclear, protection was thought to be mediated by IgM antitoxin.

The three serotypes of S. epidermidis, designated Serotype I, II, and III are based on the polysaccharide capsule of S. epidermidis. Rabbit immunization studies were conducted to determine if broadly protective antibodies to S. epidermidis were directed against multiple capsular polysaccharide serotype antigens, or against an antigen inducing broad protection across serotypes. After immunization with S. epidermidis Hay (ATCC 55133) inactivated whole cell vaccine (Figures 3 and 6), or TCA-extracted antigens, comprising surface proteins and polysaccharides (Figures 2 and 5), a rise in ELISA antibodies to TCA-extracted antigens from all serotypes was observed (Figures 2 and 3). In addition, opsonic antibodies were also induced by immunization with this single S. epidermidis strain (Figures 5 and 6).

Thus, the present invention surprisingly demonstrates that antibodies to S. epidermidis are broadly opsonic (Figures 5 and 6) and protective (Figure 9) across all three serotypes. These data demonstrate that antibodies to non-polysaccharide capsular antigens are also opsonic and provide protection against infection by S. epidermidis.

These results suggest that a surface antigen of S. epidermidis Hay (ATCC 55133) induced broadly reactive opsonic antibodies across all three S. epidermidis serotypes. Such an antigen could account for the broad protection shown in the IVIG studies. Since polysaccharide capsular antigens induce serotype specific antibodies, the present example is directed to surface proteins of S. epidermidis.

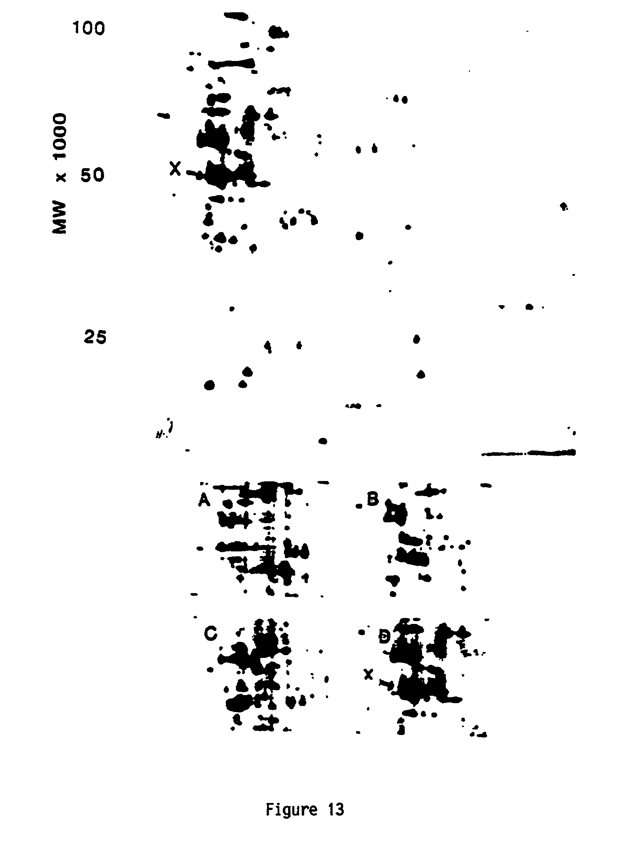

To determine the total protein composition of the various serotypes of S. epidermidis, and to identify proteins reactive with opsonic rabbit antisera, samples of each serotype were analyzed by two-dimensional gel electrophoresis. One series of gels were silver stained to visualize the component proteins of each serotype. These gels were analyzed by image processing to compare both qualitative and quantitative expression of specific proteins (Figure 13). Approximately 400 proteins could be resolved.

Another series of gels were transferred by electroblotting (Western transfer) to polyvinylidene difluoride membranes for analysis by immunodetection using the opsonic rabbit antisera to identify antigenic proteins.

### Two-Dimensional Electrophoresis:

Current 2-D technology offers the highest resolution separations available and can resolve over two thousand different proteins from highly complex cells. This level of resolution, almost two orders of magnitude greater than competing techniques, makes this technique uniquely suited to the analysis of cellular protein components. The "maps" produced by this technology result in proteins appearing as a distinct oval or round spot when detected by staining.

The analysis of S. epidermidis proteins is based upon separation and characterization by two-dimensional gel electrophoresis using the ISO-DALT system of Anderson and Anderson. The 2-D system, with slight modifications, consists of isoelectric focusing in an acrylamide gel in the first dimension followed by slab gel electrophoresis in the second dimension.

Isoelectric focusing separates proteins according to amino acid composition (primarily in relation to the ratio of acidic to basic chemical groups). Typically, a small sample of protein (100-200 *µ*g) is applied to the top of a gel formed in a 1.5 mm glass tube, and separated over 20 hours at 700 V. Low molecular weight ampholytes added to the gel generate a pH gradient within the gel. The gel rod containing isoelectrically focused proteins is removed from the tube and placed along the top edge of an acrylamide slab gel containing sodium dodecyl sulfate, an anionic detergent that unfolds each protein. The proteins migrate under the influence of an applied electrical field and separate by a sieving action according to their molecular mass. Proteins focusing between pH 3 to 10 and within the molecular mass range of 8,000 to 250,000 daltons can be resolved. A two-dimensional array of spots, each composed of a specific protein, is formed. The protein spots are then detected by staining. Radiographic methods for detection may also be used if the proteins incorporate a radioactive label.

### Identification and Purification of Proteins:

Proteins separated by two-dimensional gel electrophoresis are readily identified by immunological staining. The proteins are transferred from the acrylamide slab gel (generally prior to staining) by the method of Western blotting introduced by Towbin. This method uses a sandwich arrangement in which the proteins resolved in the acrylamide gel are electrophoretically transferred out of the gel matrix onto the surface of a membrane support, such as nitrocellulose or polyvinylidene difluoride. Proteins bound to the support can then be analyzed by immunochemical visualization reactions employing an antibody to a particular protein component. This is followed by a secondary antibody conjugated to an enzyme system, such as peroxidase or phosphatase, for visualization.

Using these techniques, one protein having a molecular weight of about 45-50,000 daltons was found to react strongly to the antisera. This protein, which focuses at a pH of approximately 4.5, is quantitatively one of the major proteins found in S. epidermidis. The protein was identified on all three S. epidermidis serotypes and in antigen preparations obtained by TCA extraction from these organisms. Figure 13 shows the separation of this protein on a two-dimensional gel, indicated by the "X" on the large picture and on panel "D."

Since the reacting protein could be extracted from whole cell bacteria by TCA, it is most likely a S. epidermidis surface protein, important for phagocytosis and immunity. A S. epidermidis protein that induced broadly reactive and protective antibodies to all serotypes of S. epidermidis is valuable as a tool for screening plasma or immunoglobulins (polyclonal or monoclonal) useful for passive immunotherapy to prevent or treat S. epidermidis infections. In addition, this protein is useful for active immunization to induce protection against S. epidermidis by vaccination. Polyclonal serum containing opsonic antibodies against S. epidermidis bound to this protein, demonstrating that this is an important surface protein of S. epidermidis that may play a significant role in the prevention and treatment of staphylococcal infections.

Antibodies to this protein are therefore broadly protective against all serotypes of S. epidermidis, and are not serotype specific, as suggested by the studies of Y. Ichiman and K. Yoshida.

### Example 16

This example provides vaccines comprising Staphylococcal antigens useful for treating and preventing Staphylococcal infections.

Table VIII shows exemplary vaccines employing various types of antigens and target organisms. As noted in the Table, several of the vaccines are conjugate vaccines. Methods of conjugation are well known to those of ordinary skill in the art, and include the heteroligation techniques of Brunswick et al., J. Immunol., 140:3364 (1988); Wong, S.S., Chemistry of Protein Conjugates and Crosslinking, CRC Press, Boston (1991); and Brenkeley et al., "Brief Survey of Methods for Preparing Protein Conjugates With Dyes, Haptens and Cross-Linking Agents," Bioconjugate Chemistry, 3, No. 1 (Jan. 1992), specifically incorporated by reference.

Other conjugate vaccines could include antigens from gram-negative or gram-positive bacteria in a variety of combinations. For example, staphylococcal polysaccharides could be conjugated to proteins from gram-negative or gram-positive bacteria, or gram-negative or gram-positive bacterial polysaccharides could be conjugated to staphylococcal proteins.

This example is not intended to be limiting, and other types of vaccines will be apparent to those skilled in the art from consideration of the specification and practice of the invention.

**TABLE VIII**

| **Vaccine** | **Type** | **Target Organisms** |
|---|---|---|
| heat-killed and | whole cell | coagulase negative |
| S. epidermidis staphylococci Hay (ATCC 55133) | | positive A |
| | | |
| surface protein 45-50,000 Daltons | purified protein | S. epidermidis (all serotypes) |
| | | |
| Serotype II polysaccharide | purified polysaccharide | human pathogenic staphylococci |
| | | |
| surface protein conjugated to Serotype II S. epidermidis polysaccharide and Serotypes 5 and VIII S. aureus polysaccharide | conjugate vaccine | human pathogenic staphylococci |
| | | |
| tetanus or diphtheria toxoid conjugated to Serotype II polysaccharide | conjugate vaccine | human pathogenic staphylococci |
| surface protein conjugated to pseudomonas polysaccharide | conjugate vaccine | all serotypes of S. epidermidis and pseudomonas (gram positive and gram negative bacteria coverage) |

It is known that both protein and polysaccharide antigens on the surface of bacteria play an important role in immunity. As provided by this invention, the 45-50 Kd surface protein of one strain of S. epidermidis induces antibodies reactive against all three serotypes of S. epidermidis. This invention also demonstrates that the Serotype II S. epidermidis capsular polysaccharide is not only protective for Serotype II S. epidermidis (Ichiman et al., J. Appl. Bacteriol., 63:165-169 (1987)), but is a common virulence marker for invasive coagulase negative staphylococci (Table VII), with all invasive strains bearing the Serotype II capsule. Thus, an organism that bears such antigens, such as Serotype II S. epidermidis Hay (ATCC 55133), is useful in isolating immunoglobulin that is both opsonic and broadly reactive (Figures 5 and 6) as well as indicating the presence of pathogenic staphylococci.

Such antigens are also useful in vaccines either alone, combined (i.e., a combination of the 45-50,000 dalton S. epidermidis surface protein and the Type II capsular polysaccharide), or combined with other important antigens. Such other antigens may include other staphylococcal capsular polysaccharides, such as Serotype 5 and Serotype 8 S. aureus capsular antigens, which are also important for inducing opsonic antibodies to staphylococci. Vaccines utilizing Serotype II polysaccharide alone are broadly reactive for coagulase negative staphylococci (CNS), but a vaccine combining Serotype II S. epidermidis and Serotypes 5 and 8 S. aureus polysaccharides would provide broadly reactive vaccine for staphylococci (coagulase negative and coagulase positive). Such vaccines would be highly immunogenic even in young infants and have broadly opsonic and protective activity for staphylococci.

To enhance immunogenicity, such polysaccharide antigens can be conjugated to proteins, such as tetanus or diphtheria toxoids, or staphylococcal proteins, as is well known in the art.

Other embodiments and uses of the invention will be apparent to those skilled in the art from consideration of the specification and practice of the invention.

## Claims

1. An isolated surface protein of a coagulase-negative staphylococcus, wherein the protein induces antibodies that are broadly reactive against staphylococci.

2. The protein of claim 1, wherein the antibodies are broadly reactive against all three serotypes of Staphylococcus epidermidis.

3. The protein of claim 1, wherein the protein is from Staphylococcus epidermidis.

4. The protein of claim 3, wherein the protein has a molecular weight of about 45,000 to 50,000 daltons, and has an isoelectric focusing point of approximately pH 4.5.

5. The protein of claim 3 or 4, wherein the Staphylococcus epidermidis is strain Hay deposited at the A.T.C.C. under Accession No. 55133.

6. Isolated opsonic antibody against a surface protein of a coagulase-negative staphylococcus, wherein the protein induces antibodies that are broadly reactive against staphylococci.

7. Antibody of claim 6, wherein the antibody is monoclonal or polyclonal antibody.

8. Antibody of claim 6, wherein the antibody is broadly reactive against all three serotypes of Staphylococcus epidermidis.

9. Antibody of claim 6, wherein the protein is from Staphylococcus epidermidis.

10. Antibody of claim 9, wherein the protein has a molecular weight of about 45,000 to 50,000 daltons, and has an isoelectric focusing point of approximately pH 4.5.

11. Antibody of claim 9 or 10, wherein the Staphylococcus epidermidis is strain Hay deposited at the A.T.C.C. under Accession No. 55133.

12. A method of isolating broadly reactive and opsonic immunoglobulin useful for the treatment and prevention of a staphylococcal infection comprising:
(a) providing a sample of immunoglobulin;
(b) assaying the immunoglobulin for the presence of antibody against a surface protein of a coagulase-negative staphylococcus, wherein the protein induces antibodies that are broadly reactive against staphylococci; and
(c) isolating immunoglobulin having a high titer of said antibody;
wherein the presence of antibody against said protein is indicative of the usefulness of the biological fluid for the treatment and prevention of the staphylococcal infections.

13. The method of claim 12, wherein the protein is from Staphylococcus epidermidis.

14. The method of claim 13, wherein the protein has a molecular weight of about 45,000 to 50,000 daltons and an isoelectric focusing point of approximately pH 4.5.

15. The method of claim 13 or 14, wherein the protein is from Staphylococcus epidermidis strain Hay deposited at the A.T.C.C. under Accession No. 55133.

16. The method of claim 12, wherein the immunoglobulin comprises polyclonal or monoclonal antibodies.

17. The method of claim 12, wherein the assay is selected from the group consisting of a binding assay, an opsonization assay, and a clearance assay.

18. The method of claim 17, wherein the opsonic assay is a cell mediated bactericidal assay.

19. A pharmaceutical composition for the treatment or prevention of staphylococcal infections comprising:
(a) opsonic antibody against a surface protein from a coagulase-negative staphylococcus, wherein the antibody is broadly reactive against staphylococci; and
(b) a pharmaceutically acceptable carrier.

20. The composition of claim 19, wherein the protein is from Staphylococcus epidermidis.

21. The composition of claim 20, wherein the protein has a molecular weight of about 45,000 to 50,000 daltons and an isoelectric focusing point of approximately pH 4.5.

22. The composition of claim 20 or 21, wherein the protein is from Staphylococcus epidermidis strain Hay deposited at the A.T.C.C. under Accession No. 55133.

23. The composition of claim 19, wherein the pharmaceutically acceptable carrier is selected from the group consisting of water, oils, saline solutions, aqueous dextrose, and glycerol solutions.

24. A vaccine for the treatment or prevention of staphylococcal infections comprising:
(a) a therapeutically effective amount of a surface protein from a coagulase-negative staphylococcus, which protein induces antibodies that are broadly reactive against staphylococci; and
(b) a pharmaceutically acceptable carrier.

25. The vaccine of claim 24, wherein the protein is from Staphylococcus epidermidis.

26. The vaccine of claim 25, wherein the protein has a molecular weight of about 45,000 to 50,000 daltons and an isoelectric focusing point of approximately pH 4.5.

27. The vaccine of claim 25 or 26, wherein the protein is from Staphylococcus epidermidis strain Hay deposited at the A.T.C.C. under Accession No. 55133.

28. The vaccine of claim 24, wherein the pharmaceutically acceptable carrier is selected from the group consisting of water, oils, saline solutions, aqueous dextrose, and glycerol solutions.

29. The vaccine of claim 24, wherein the protein is conjugated to a second compound.

30. The vaccine of claim 29, wherein the second compound is a capsular polysaccharide of S. aureus Serotype 5, S. aureus Serotype 8, or S. epidermidis.

31. The vaccine of claim 29, wherein the second compound is a capsular polysaccharide of a gram-negative organism.

32. The vaccine of claim 24, wherein the vaccine is conjugated to a surface protein from a coagulase-negative staphylococcus, which protein induces antibodies that are broadly reactive against staphylococci.

33. The vaccine of claim 32, wherein the surface protein has a molecular weight of about 45,000 to 50,000 daltons and an isoelectric focusing point of approximately pH 4.5.

34. The vaccine of claim 32 or 33, wherein the polysaccharide and the protein are from Staphylococcus epidermidis strain Hay deposited at the A.T.C.C. under Accession No. 55133.

35. A method of isolating broadly reactive and opsonic immunoglobulin useful for the treatment and prevention of a staphylococcal infection comprising:
(a) providing a sample of immunoglobulin;
(b) assaying the immunoglobulin for the presence of antibody against a Serotype II capsular polysaccharide antigen of a coagulase-negative staphylococcus, wherein the antigen induces antibodies broadly reactive against staphylococci;
(c) assaying the immunoglobulin for the presence of antibody against a surface protein antigen of a coagulase-negative staphylococcus, wherein the protein induces antibodies that are broadly reactive against staphylococci; and
(d) isolating immunoglobulin having a high titer of said antibody;
wherein the presence of antibody against said antigens is indicative of the usefulness of the biological fluid for the treatment and prevention of staphylococcal infections.

36. The method of claim 35, wherein the antigens are from Staphylococcus epidermidis.

37. The method of claim 35, wherein the protein antigen is from Staphylococcus epidermidis and has a molecular weight of about 45,000 to 50,000 daltons and an isoelectric focusing point of approximately pH 4.5.

38. The method of claim 36 or 37, wherein the Staphylococcus epidermidis is strain Hay deposited at the A.T.C.C. under Accession No. 55133.

39. A method of isolating broadly reactive and opsonic immunoglobulin useful for the treatment and prevention of a staphylococcal infection comprising:
(a) providing a sample of immunoglobulin;
(b) assaying the immunoglobulin for the presence of antibody against a Serotype II coagulase-negative staphylococcus bearing a surface protein antigen, or a combination thereof, wherein the antigens induce opsonic antibodies that are broadly reactive against staphylococci; and
(c) isolating immunoglobulin having a high titer of said antibody;
wherein the presence of antibody against said staphylococcus is indicative of the usefulness of the biological fluid for the treatment and prevention of staphylococcal infections.

40. The method of claim 39, wherein the staphylococcus is Staphylococcus epidermidis.

41. The method of claim 40, wherein the Staphylococcus epidermidis is strain Hay deposited at the A.T.C.C. under Accession No. 55133.

## Patentansprüche

1. Isoliertes Oberflächenprotein eines Koagulase-negativen Staphylococcus, wobei das Protein Antikörper induziert, die gegen Staphylokokken breitreaktiv sind.

2. Protein gemäß Anspruch 1, wobei die Antikörper gegen alle drei Serotypen von *Staphylococcus epidermidis* breitreaktiv sind.

3. Protein gemäß Anspruch 1, wobei das Protein aus *Staphylococcus epidermidis* stammt.

4. Protein gemäß Anspruch 3, wobei das Protein ein Molekulargewicht von etwa 45.000 bis 50.000 Dalton und einen isoelektrischen Fokussierungspunkt bei einem pH-Wert von etwa 4,5 hat.

5. Protein gemäß Anspruch 3 oder 4, wobei es sich bei dem *Staphylococcus epidermidis* um den bei der A.T.C.C. unter der Zugriffs-Nr. 55133 hinterlegten Stamm Hay handelt.

6. Isolierter opsonischer Antikörper gegen ein Oberflächenprotein eines Koagulase-negativen Staphylokokkus, wobei das Protein Antikörper induziert, die gegen Staphylokokken breitreaktiv sind.

7. Antikörper gemäß Anspruch 6, wobei es sich bei dem Antikörper um einen monoklonalen oder polyklonalen Antikörper handelt.

8. Antikörper gemäß Anspruch 6, wobei der Antikörper gegen alle drei Serotypen von *Staphylococcus epidermidis* breitreaktiv ist.

9. Antikörper gemäß Anspruch 6, wobei das Protein von *Staphylococcus epidermidis* stammt.

10. Antikörper gemäß Anspruch 9, wobei das Protein ein Molekulargewicht von etwa 45.000 bis 50.000 Dalton und einen isoelektrischen Fokussierungspunkt bei einem pH-Wert von etwa 4,5 hat.

11. Antikörper gemäß Anspruch 9 oder 10, wobei es sich bei dem *Staphylococcus epidermidis* um den bei der A.T.C.C. unter der Zugriffs-Nr. 55133 hinterlegten Stamm Hay handelt.

12. Verfahren zur Isolierung von breitreaktivem und opsonischem Immunglobulin, das zur Behandlung einer Staphylokokken-Infektion oder zur Vorbeugung dagegen verwendbar ist, umfassend:
a) Bereitstellen einer Immunglobulinprobe,
b) Testen des Immunglobulins auf das Vorhandensein eines Antikörpers gegen ein Oberflächenprotein eines Koagulase-negativen Staphylococcus, wobei das Protein Antikörper induziert, die gegen Staphylokokken breitreaktiv sind, und
c) Isolieren von Immunglobulin mit hohem Titer des Antikörpers,
wobei das Vorhandensein von Antikörper gegen das Protein ein Hinweis auf die Verwendbarkeit der biologischen Flüssigkeit zur Behandlung von Staphylokokken-Infektionen oder zur Vorbeugung dagegen ist.

13. Verfahren gemäß Anspruch 12, in dem das Protein von *Staphylococcus epidermidis* stammt.

14. Verfahren gemäß Anspruch 13, in dem das Protein ein Molekulargewicht von etwa 45.000 bis 50.000 Dalton und einen isoelektrischen Fokussierungspunkt bei einem pH-Wert von etwa 4,5 hat.

15. Verfahren gemäß Anspruch 13 oder 14, in dem das Protein von dem bei der A.T.C.C. unter der Zugriffs-Nr. 55133 hinterlegten Stamm Hay von *Staphylococcus epidermidis* stammt.

16. Verfahren gemäß Anspruch 12, in dem das Immunglobulin polyklonale oder monoklonale Antikörper umfasst.

17. Verfahren gemäß Anspruch 12, in dem der Test aus der Gruppe bestehend aus einem Bindungstest, einem Opsonierungstest und einem Clearance-Test ausgewählt wird.

18. Verfahren gemäß Anspruch 17, in dem es sich bei dem Opsonierungstest um einen Test der zelivermittelten bakteriziden Aktivität handelt.

19. Pharmazeutische Zusammensetzung zur Behandlung von Staphylokokken-Infektionen oder zur Vorbeugung dagegen, umfassend:
a) opsonischen Antikörper gegen ein Oberflächenprotein von einem Koagulase-negativen Staphylokokkus, wobei der Antikörper gegen Staphylokokken breitreaktiv ist, und
b) einen pharmazeutisch annehmbaren Träger.

20. Zusammensetzung gemäß Anspruch 19, in der das Protein von *Staphylococcus epidermidis* stammt.

21. Zusammensetzung gemäß Anspruch 20, in der das Protein ein Molekulargewicht von etwa 45.000 bis 50.000 Dalton und einen isoelektrischen Fokussierungspunkt bei einem pH-Wert von etwa 4,5 hat.

22. Zusammensetzung gemäß Anspruch 20 oder 21, in der das Protein von dem bei der A.T.C.C. unter der Zugriffs-Nr. 55133 hinterlegten Stamm Hay von *Staphylococcus epidermidis* stammt.

23. Zusammensetzung gemäß Anspruch 19, in der der pharmazeutisch annehmbare Träger aus der Gruppe bestehend aus Wasser, Ölen, Kochsalzlösungen, wässrigen Dextroselösungen und Glycerinlösungen ausgewählt ist.

24. Impfstoff zur Behandlung von Staphylokokken-Infektionen oder zur Vorbeugung dagegen, umfassend:
a) eine therapeutisch wirksame Menge eines Oberflächenproteins von einem Koagulase-negativen Staphylokokkus, wobei das Protein Antikörper induziert, die gegen Staphylokokken breitreaktiv sind, und
b) einen pharmazeutisch annehmbaren Träger.

25. Impfstoff gemäß Anspruch 24, in dem das Protein von *Staphylococcus epidermidis* stammt.

26. Impfstoff gemäß Anspruch 25, in dem das Protein ein Molekulargewicht von etwa 45.000 bis 50.000 Dalton und einen isoelektrischen Fokussierungspunkt bei einem pH-Wert von etwa 4,5 hat.

27. Impfstoff gemäß Anspruch 25 oder 26, in dem das Protein von dem bei der A.T.C.C. unter der Zugriffs-Nr. 55133 hinterlegten Stamm Hay von *Staphylococcus epidermidis* stammt.

28. Impfstoff gemäß Anspruch 24, in dem der pharmazeutisch annehmbare Träger aus der Gruppe bestehend aus Wasser, Ölen, Kochsalzlösungen, wässrigen Dextroselösungen und Glycerinlösungen ausgewählt ist.

29. Impfstoff gemäß Anspruch 24, in dem das Protein mit einer zweiten Verbindung konjugiert ist.

30. Impfstoff gemäß Anspruch 29, in dem es sich bei der zweiten Verbindung um ein Kapselpolysaccharid von *S*. *aureus* Serotyp 5, *S. aureus* Serotyp 8 oder *S. epidermidis* handelt.

31. Impfstoff gemäß Anspruch 29, in dem es sich bei der zweiten Verbindung um ein Kapselpolysaccharid eines gramnegativen Organismus handelt.

32. Impfstoff gemäß Anspruch 24, in dem der Impfstoff mit einem Oberflächenprotein von einem Koagulase-negativen Staphylokokkus konjugiert ist, wobei das Protein Antikörper induziert, die gegen Staphylokokken breitreaktiv sind.

33. Impfstoff gemäß Anspruch 32, in dem das Protein ein Molekulargewicht von etwa 45.000 bis 50.000 Dalton und einen isoelektrischen Fokussierungspunkt bei einem pH-Wert von etwa 4,5 hat.

34. Impfstoff gemäß Anspruch 32 oder 33, in dem das Polysaccharid und das Protein von dem bei der A.T.C.C. unter der Zugriffs-Nr. 55133 hinterlegten Stamm Hay von *Staphylococcus epidermidis* stammen.

35. Verfahren zur Isolierung von breitreaktivem und opsonischem Immunglobulin, das zur Behandlung einer Staphylokokken-Infektion oder zur Vorbeugung dagegen verwendbar ist, umfassend:
a) Bereitstellen einer Immunglobulinprobe,
b) Testen des Immunglobulins auf das Vorhandensein von Antikörper gegen ein Serotyp II-Kapselpolysaccharid-Antigen eines Koagulase-negativen Staphylokokkus, wobei das Antigen Antikörper induziert, die gegen Staphylokokken breitreaktiv sind,
c) Testen des Immunglobulins auf das Vorhandensein von Antikörper gegen ein Oberflächenprotein-Antigen eines Koagulase-negativen Staphylokokkus, wobei das Protein Antikörper induziert, die gegen Staphylokokken breitreaktiv sind, und
d) Isolieren von Immunglobulin mit hohem Titer des Antikörpers,
wobei das Vorhandensein von Antikörper gegen die Antigene ein Hinweis auf die Verwendbarkeit der biologischen Flüssigkeit zur Behandlung von Staphylokokken-Infektionen oder zur Vorbeugung dagegen ist.

36. Verfahren gemäß Anspruch 35, in dem die Antigene von *Staphylococcus epidermidis* stammen.

37. Verfahren gemäß Anspruch 35, in dem das Proteinantigen von *Staphylococcus epidermidis* stammt und ein Molekulargewicht von etwa 45.000 bis 50.000 Dalton und einen isoelektrischen Fokussierungspunkt bei einem pH-Wert von etwa 4,5 hat.

38. Verfahren gemäß Anspruch 36 oder 37, in dem es sich bei dem *Staphylococcus epidermidis* um den bei der A.T.C.C. unter der Zugriffsnummer 55133 hinterlegten Stamm Hay handelt.

39. Verfahren zur Isolierung von breitreaktivem und opsonischem Immunglobulin, das zur Behandlung einer Staphylokokken-Infektion oder zur Vorbeugung dagegen verwendbar ist, umfassend:
a) Bereitstellen einer Immunglobulinprobe,
b) Testen des Immunglobulins auf das Vorhandensein von Antikörper gegen ein Koagulase-negatives Staphylokokkus von Serotyp II, das ein Oberflächenproteinantigen oder eine Kombination davon besitzt, wobei die Antigene opsonische Antikörper induzieren, die gegen Staphylokokken breitreaktiv sind, und
c) Isolieren von Immunglobulin mit hohem Titer des Antikörpers,
wobei das Vorhandensein von Antikörper gegen das Staphylokokkus ein Hinweis auf die Verwendbarkeit der biologischen Flüssigkeit zur Behandlung von Staphylokokken-Infektionen oder zur Vorbeugung dagegen ist.

40. Verfahren gemäß Anspruch 39, in dem es sich bei dem Staphylokokkus um *Staphylococcus epidermidis* handelt.

41. Verfahren gemäß Anspruch 40, in dem es sich bei dem *Staphylococcus epidermidis* um den bei der A.T.C.C. unter der Zugriffs-Nr. 55133 hinterlegten Stamm Hay handelt.

## Revendications

1. Protéine de surface isolée d'un staphylocoque coagulase-négatif, dans laquelle la protéine induit des anticorps qui sont réactifs avec un large spectre Contre des staphylocoques.

2. Protéine de la revendication 1, dans laquelle les anticorps sont réactifs avec un large spectre contre tous les trois sérotypes de *Staphylococcus epidermidis*.

3. Protéine de la revendication 1, dans laquelle la protéine est issue de *Staphylococcus epidermidis.*

4. Protéine de la revendication 3, dans laquelle la protéine a une masse moléculaire d'environ 45 000 à 50 000 daltons et a un point de focalisation isoélectrique d'environ pH 4,5.

5. Protéine de la revendication 3 ou 4, dans laquelle le *Staphylococcus epidermidis* est la souche Hay déposée à l'ATCC sous le n° de dépôt 55133.

6. Anticorps à activité d'opsonine isolé, contre une protéine de surface d'un staphylocoque coagulase-négatif, dans lequel la protéine induit des anticorps qui sont réactifs avec un large spectre contre des staphylocoques.

7. Anticorps de la revendication 8, dans lequet l'anticorps est un anticorps monoclonal ou polyclonal.

8. Anticorps de la revendication 6, dans lequel l'anticorps est réactif avec un large spectre contre tous les trois sérotypes de *Staphylococcus epidermidis.*

9. Anticorps de la revendication 6, dans lequel la protéine est issue de *Staphylococcus epidermidis.*

10. Anticorps de la revendication 9, dans lequel la protéine a une masse moléculaire d'environ 45 000 à 50 000 daltons et a un point de focalisation isoélectrique d'environ pH 4,5.

11. Anticorps de la revendication 9 ou 10, dans lequel le *Staphylococcus epidermidis* est la souche Hay déposée à l'ATCC sous le n° de dépôt 55133.

12. Procédé d'isolement d'une immunoglobuline réactive avec un large spectre et à activité d'opsonine, utile pour le traitement et la prévention d'une infection à staphylocoques, comprenant:
(a) la production d'un échantillon d'immunoglobuline ;
(b) le dosage de l'immunoglobuline quant à la présence d'un anticorps dirigé contre une protéine de surface d'un staphylocoque coagulase-négatif, dans lequel la protéine induit des anticorps qui sont réactifs avec un large spectre contre des staphylocoques ; et
(c) l'isolement d'immunoglobuline ayant un titre élevé dudit anticorps ;
dans lequel la présence d'anticorps contre ladite protéine indique la possibilité d'utilisation du liquide biologique pour le traitement et la prévention des infections à staphylocoques.

13. Procédé de la revendication 12, dans lequel la protéine est issue de *Staphylococcus epldermidis.*

14. Procédé de la revendication 13, dans lequel la protéine a une masse moléculaire d'environ 45 000 à 50 000 daltons et a un point de focalisation isoélectrique d'environ pH 4,5.

15. Procédé de la revendication 13 ou 14, dans lequel la protéine est issue de la souche Hay de *Staphylococcus epidermidis* déposée à l'ATCC sous le n° de dépôt 55133.

16. Procédé de la revendication 12, dans lequel l'immunoglobullne comprend des anticorps polyclonaux ou monoclonaux.

17. Procédé de la revendication 12, dans lequel le dosage est choisi dans l'ensemble constitué par un essai de liaison, un essai d'opsonisation et un essai de clairance.

18. Procédé de la revendication 17, dans lequel l'essai d'opsonisation est un essai bactéricide à médiation cellulaire.

19. Composition pharmaceutique pour le traitement ou la prévention d'infections à staphylocoques, comprenant :
(a) un anticorps à activité d'opsonine dirigé contre une protéine de surface issue d'un staphylocoque coagulase-négatif, l'anticorps étant réactif avec un large spectre contre des staphylocoques : et
(b) un véhicule pharmaceutiquement acceptable.

20. Composition de la revendication 19, dans laquelle la protéine est Issue de *Staphylococcus epidermidis.*

21. Composition de la revendication 20, dans laquelle la protéine a une masse moléculaire d'environ 45 000 à 50 000 daltons et a un point de focalisation isoélectrique d'environ pH 4,5.

22. Composition de la revendication 20 ou 21, dans laquelle la protéine est Issue de la souche Hay de *Staphylococcus epidermidis* déposée à l'ATCC sous le n° de dépôt 55133.

23. Composition de la revendication 19, dans laquelle le véhicule pharmaceutiquement acceptable est choisi dans l'ensemble constitué par l'eau, des huiles, des solutions de chlorure de sodium, une solution aqueuse de glucose et des solutions de glycérol.

24. Vaccin pour le traitement ou la prévention d'infections à staphylocoques, comprenant :
(a) une quantité thérapeutiquement efficace d'une protéine de surface issue d'un staphylocoque coagulase-négatif, laquelle protéine induit des anticorps qui sont réactifs avec un large spectre contre des staphylocoques ; et
(b) un véhicule pharmaceutiquement acceptable.

25. Vaccin de la revendication 24, dans lequel la protéine est issue de *Staphylococcus epidermidis.*

26. Vaccin de la revendication 25, dans lequel la protéine a une masse moléculaire d'environ 45 000 à 50 000 daltons et a un point de focalisation isoélectrique d'environ pH 4,5.

27. Vaccin de la revendication 25 ou 26, dans lequel la protéine est issue de la souche Hay de *Staphylococcus epidermidis* déposée à l'ATCC sous le n° de dépôt 55133.

28. Vaccin de la revendication 24, dans lequel le véhicule pharmaceutiquement acceptable est choisi dans l'ensemble constitué par l'eau, des huiles, des solutions de chlorure de sodium, une solution aqueuse de glucose et des solutions de glycérol.

29. Vaccin de la revendication 24, dans lequel la protéine est conjuguée à un second composé.

30. Vaccin de la revendication 29, dans lequel le second composé est un polysaccharide de capsule de *S. aureus* sérotype 5, *S. aureus* sérotype 8 ou *S. epidermidis*.

31. Vaccin de la revendication 29, dans lequel le second composé est un polysaccharide de capsule d'un organisme à Gram négatif.

32. Vaccin de la revendication 24, dans lequel le vaccin est conjugué à une protéine de surface Issue d'un staphylocoque coagulase-négatif, laquelle protéine induit des anticorps qui sont réactifs avec un large spectre contre des staphylocoques.

33. Vaccin de la revendication 32, dans lequel la protéine de surface à une masse moléculaire d'environ 45 000 à 60 000 daltons et a un point de focallsation isoélectrique d'environ pH 4,5.

34. Vaccin de la revendication 32 ou 33, dans lequel le polysaccharide et la protéine sont issus de la souche Hay de *Staphylococcus epidermidis* déposée à l'ATCC sous le n° de dépôt 55133.

35. Procédé d'isolement d'une immunoglobuline réactive avec un large spectre et à activité d'opsonine, utile pour le traitement et la prévention d'une infection à staphylocoques, comprenant :
(a) la production d'un échantillon d'immunoglobuline ;
(b) le dosage de l'immunoglobuline quant à la présence d'un anticorps dirigé contre un antigène polysaccharidique de capsule de sérotype II ou d'un staphylocoque coagulase-négatif, dans lequel l'antigène induit des anticorps qui sont réactifs avec un large spectre contre des staphylocoques ; et
(c) le dosage de l'immunoglobuline quant à la présence d'un anticorps dirigé contre un antigène protéique de surface d'un staphylocoque coagulase-négatif, dans lequel la protéine induit des anticorps qui sont réactifs avec un large spectre contre des staphylocoques ; et
(d) l'isolement d'immunoglobuline ayant un titre élevé dudit anticorps ;
dans lequel la présence d'anticorps contre lesdits antigènes indique la possibilité d'utilisation du liquide biologique pour le traitement et la prévention d'infections à staphylocoques.

36. Procédé de la revendication 35, dans lequel les antigènes sont issus de *Staphylococcus epidermidis*.

37. Procédé de la revendication 35, dans lequel l'antigène protéique est issu de *Staphylococcus epidermidis* et a une masse moléculaire d'environ 45 000 à 50 000 daltons et un point de focalisation isoélectrique d'environ pH 4,5.

38. Procédé de la revendication 36 ou 37, dans lequel le *Staphylococcus epidermidis* est la souche Hay déposée à l'ATCC sous le n° de dépôt 55133.

39. Procédé d'isolement d'une immunoglobuline réactive avec un large spectre et à activité d'opsonine, utile pour le traitement et la prévention d'une infection à staphylocoques, comprenant :
(a) la production d'un échantillon d'immunoglobuline ;
(b) le dosage de l'immunoglobuline quant à la présence d'un anticorps dirigé contre un staphylocoque coagulase-négatif de sérotype II, portant un antigène protéique de surface, ou une association de tels antigènes, dans lequel les antigènes induisent des anticorps qui sont réactifs avec un large spectre contre des staphylocoques ; et
(c) l'isolement d'immunoglobuline ayant un titre élevé dudit anticorps ;
dans lequel la présence d'anticorps contre ledit staphylocoque indique la possibilité d'utilisation du liquide biologique pour le traitement et la prévention d'infections à staphylocoques.

40. Procédé de la revendication 39, dans lequel le staphylocoque est *Staphylococcus epidermidis.*

41. Procédé de la revendication 40, dans lequel le *Staphylococcus epidermidis* est la souche Hay déposée à l'ATCC sous le n° de dépôt 55133.
